(19) Europäisches Patentamt
European Patent Office
Office européen des brevets

(11) EP 4 729 615 A1

(12) EUROPEAN PATENT APPLICATION
published in accordance with Art. 153(4) EPC

(43) Date of publication:
**22.04.2026 Bulletin 2026/17**

(21) Application number: **24822708.4**

(22) Date of filing: **12.06.2024**

(51) International Patent Classification (IPC):
***C12N 15/113*** (2010.01) ***A61K 31/713*** (2006.01)
***A61K 31/712*** (2006.01) ***A61K 31/7125*** (2006.01)
***A61K 38/00*** (2006.01) ***A61P 11/00*** (2006.01)
***A61P 35/00*** (2006.01)

(52) Cooperative Patent Classification (CPC):
**A61K 31/712; A61K 31/7125; A61K 31/713; A61K 38/00; A61P 11/00; A61P 35/00; C12N 15/113**

(86) International application number:
**PCT/CN2024/098635**

(87) International publication number:
**WO 2024/255759 (19.12.2024 Gazette 2024/51)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **13.06.2023 CN 202310696482**
**18.01.2024 CN 202410074824**

(71) Applicant: **Tuojie Biotech (Shanghai) Co., Ltd.**
**Shanghai 201203 (CN)**

(72) Inventors:
- **LIU, Min**
  **Shanghai 201203 (CN)**
- **LI, Yunfei**
  **Shanghai 201203 (CN)**
- **LIN, Xiaoyan**
  **Shanghai 201203 (CN)**
- **WANG, Yanhui**
  **Shanghai 201203 (CN)**

(74) Representative: **Potter Clarkson**
**Chapel Quarter**
**Mount Street**
**Nottingham NG1 6HQ (GB)**

Remarks:
The complete document including Reference Table(s) and the Sequence Listing(s) can be downloaded from the EPO website

# (54) SIRNA TARGETING MMP7, SIRNA CONJUGATE AND MEDICAL USE THEREOF

(57) The present invention relates to an siRNA targeting MMP7, an siRNA conjugate and the medical use thereof. The present invention also relates to a pharmaceutic al composition comprising the siRNA, a cell or a kit, and a method for treating and/or preventing a subject suffering from related disorders by using the siRNA and the siRNA conjugate.

EP 4 729 615 A1

## Description

The present disclosure claims priority to Chinese Application No. 202310696482.8 filed on Jun. 13, 2023 and Chinese Application No. 202410074824.7 filed on Jan. 18, 2024, which are incorporated herein by reference in their entirety.

## TECHNICAL FIELD

The present disclosure pertains to the field of biomedicine, and specifically relates to an siRNA targeting matrix metalloproteinase 7 (MMP7), a conjugate, a composition, and medical use thereof.

## BACKGROUND

Matrix metalloproteinase 7 (MMP7) is the smallest member (28 kDa) of the metalloproteinase family (MMPs). MMPs comprise 23 family members with different substrates and functions, and can degrade all components of the extracellular matrix (e.g., elastin, proteoglycan, type IV collagen, fibronectin, etc.) and many non-matrix proteins such as cytokines. These functional roles in extracellular matrix remodeling and cytokine signaling regulation link MMP family members to common pathogenic mechanisms leading to cancer, chronic inflammation, and fibrosis. MMP7 is mainly expressed and secreted by epithelial cells of organs in the whole body and plays an important role in epithelial cell repair. Increased MMP7 expression is associated with pathogenic fibrosis of the lung, liver, and kidney. MMP7 gene and protein expression levels are significantly elevated in the lung tissues of idiopathic pulmonary fibrosis (IPF) and are also significantly up-regulated in the bronchoalveolar lavage (BAL) fluid. Serum MMP7 expression serves as a potent IPF serum biomarker, correlating with the severity and progression of IPF.

MMP7 is associated with pathogenic fibrosis through a variety of potential mechanisms, including promotion of epithelial-mesenchymal transition (EMT), degradation of extracellular matrix, abnormal matrix repair, and tissue remodeling. MMP7 promotes fibrosis by cleaving E-cadherin to activate epithelial cells and proteolytically activate heparin-binding epidermal growth factor-like growth factor precursor (pro-HB-EGF) to release active HB-EGF. Studies have shown that MMP7 gene-knockout mice are protected from bleomycin-induced pulmonary fibrosis, exhibiting reduced lung inflammation, fibrosis, and mortality. This suggests that MMP7 knockdown has a therapeutic effect on pulmonary fibrosis.

## SUMMARY

The present disclosure provides an siRNA. In some embodiments, the present disclosure provides an siRNA targeting MMP7, which comprises a sense strand and an antisense strand forming a double-stranded region.

In some embodiments, the sense strand comprises a sequence differing from any one of the nucleotide sequences of SEQ ID NO: 1 to SEQ ID NO: 10 and SEQ ID NO: 61 to SEQ ID NO: 151 by no more than 3 (e.g., 0, 1, 2, or 3) nucleotides and comprises at least 15 (e.g., 16, 17, 18, 19, 20, or 21) contiguous nucleotides;

the antisense strand comprises a sequence differing from any one of the nucleotide sequences of SEQ ID NO: 31 to SEQ ID NO: 40 and SEQ ID NO: 152 to SEQ ID NO: 242 by no more than 3 (e.g., 0, 1, 2, or 3) nucleotides and comprises at least 15 (e.g., 16, 17, 18, 19, 20, or 21) contiguous nucleotides.

In some embodiments, the antisense strand is at least partially reverse complementary to a target sequence to mediate RNA interference. In some embodiments, there are no more than 5, no more than 4, no more than 3, no more than 2, or no more than 1 mismatch between the antisense strand and the target sequence. In some embodiments, the antisense strand is fully reverse complementary to the target sequence.

In some embodiments, the sense strand is at least partially reverse complementary to the antisense strand to form a double-stranded region. In some embodiments, there are no more than 5, no more than 4, no more than 3, no more than 2, or no more than 1 mismatch between the sense strand and the antisense strand. In some embodiments, the sense strand is fully reverse complementary to the antisense strand.

In some embodiments, the siRNA of the present disclosure comprises one or two blunt ends.

In some embodiments, each strand of the siRNA independently comprises an overhang formed by 1 to 2 unpaired nucleotides.

In some embodiments, the siRNA of the present disclosure comprises an overhang at the 3' end of the antisense strand of the siRNA.

In some embodiments, the sense strand and the antisense strand each independently have 16 to 35, 16 to 34, 17 to 34, 17 to 33, 18 to 33, 18 to 32, 18 to 31, 18 to 30, 18 to 29, 18 to 28, 18 to 27, 18 to 26, 18 to 25, 18 to 24, 18 to 23, 19 to 25, 19 to 24, or 19 to 23 nucleotides (e.g., 19, 20, 21, 22, or 23 nucleotides).

In some embodiments, the sense strand and the antisense strand are identical or different in length; the sense strand is 19-23 nucleotides in length, and the antisense strand is 19-26 nucleotides in length. The length ratio of the sense

strand to the antisense strand of the siRNA provided by the present disclosure may be 19/19, 19/20, 19/21, 19/22, 19/23, 19/24, 19/25, 19/26, 20/19, 20/20, 20/21, 20/22, 20/23, 20/24, 20/25, 20/26, 21/20, 21/21, 21/22, 21/23, 21/24, 21/25, 21/26, 22/20, 22/21, 22/22, 22/23, 22/24, 22/25, 22/26, 23/20, 23/21, 23/22, 23/23, 23/24, 23/25, or 23/26. In some embodiments, the length ratio of the sense strand to the antisense strand of the siRNA is 19/21, 21/23, or 23/25.

In some specific embodiments, the length ratio of the sense strand to the antisense strand of the siRNA is 19/21.

In some embodiments, the sense strand comprises at least 15 contiguous nucleotides and differs from any one of the nucleotide sequences of SEQ ID NO: 1 to SEQ ID NO: 10 and SEQ ID NO: 61 to SEQ ID NO: 151 by no more than 2 nucleotides. In some embodiments, the nucleotide sequence difference is no more than 1 nucleotide; in some embodiments, the difference is 1 nucleotide.

In some embodiments, the antisense strand comprises at least 15 contiguous nucleotides and differs from any one of the nucleotide sequences of SEQ ID NO: 31 to SEQ ID NO: 40 and SEQ ID NO: 152 to SEQ ID NO: 242 by no more than 2 nucleotides; in some embodiments, the nucleotide sequence difference is no more than 1 nucleotide; in some embodiments, the difference is 1 nucleotide.

In some embodiments, the sense strand comprises at least 15 contiguous nucleotides of any one of the nucleotide sequences of SEQ ID NO: 1 to SEQ ID NO: 10 and SEQ ID NO: 61 to SEQ ID NO: 151. In some embodiments, the sense strand comprises at least 16 contiguous nucleotides of any one of the nucleotide sequences of SEQ ID NO: 1 to SEQ ID NO: 10 and SEQ ID NO: 61 to SEQ ID NO: 151. In some embodiments, the sense strand comprises at least 17 contiguous nucleotides of any one of the nucleotide sequences of SEQ ID NO: 1 to SEQ ID NO: 10 and SEQ ID NO: 61 to SEQ ID NO: 151. In some embodiments, the sense strand comprises at least 19 contiguous nucleotides of any one of the nucleotide sequences of SEQ ID NO: 1 to SEQ ID NO: 10 and SEQ ID NO: 61 to SEQ ID NO: 151.

In some embodiments, the antisense strand comprises at least 15 contiguous nucleotides of any one of the nucleotide sequences of SEQ ID NO: 31 to SEQ ID NO: 40 and SEQ ID NO: 152 to SEQ ID NO: 242. In some embodiments, the antisense strand comprises at least 17 contiguous nucleotides of any one of the nucleotide sequences of SEQ ID NO: 31 to SEQ ID NO: 40 and SEQ ID NO: 152 to SEQ ID NO: 242. In some embodiments, the antisense strand comprises at least 19 contiguous nucleotides of any one of the nucleotide sequences of SEQ ID NO: 31 to SEQ ID NO: 40 and SEQ ID NO: 152 to SEQ ID NO: 242. In some embodiments, the antisense strand comprises at least 20 contiguous nucleotides of any one of the nucleotide sequences of SEQ ID NO: 31 to SEQ ID NO: 40 and SEQ ID NO: 152 to SEQ ID NO: 242. In some embodiments, the antisense strand comprises at least 21 contiguous nucleotides of any one of the nucleotide sequences of SEQ ID NO: 31 to SEQ ID NO: 40 and SEQ ID NO: 152 to SEQ ID NO: 242.

In some embodiments, the sense strand comprises or is selected from the group consisting of any one of the following nucleotide sequences: SEQ ID NO: 1 to SEQ ID NO: 10 and SEQ ID NO: 61 to SEQ ID NO: 151.

In some embodiments, the antisense strand comprises or is selected from the group consisting of any one of the following nucleotide sequences: SEQ ID NO: 31 to SEQ ID NO: 40 and SEQ ID NO: 152 to SEQ ID NO: 242.

In some embodiments, the siRNA comprises or is selected from any one of the following groups:

group 1), a sense strand set forth in SEQ ID NO: 1 and an antisense strand set forth in SEQ ID NO: 31;
group 2), a sense strand set forth in SEQ ID NO: 2 and an antisense strand set forth in SEQ ID NO: 32;
group 3), a sense strand set forth in SEQ ID NO: 3 and an antisense strand set forth in SEQ ID NO: 33;
group 4), a sense strand set forth in SEQ ID NO: 4 and an antisense strand set forth in SEQ ID NO: 34;
group 5), a sense strand set forth in SEQ ID NO: 5 and an antisense strand set forth in SEQ ID NO: 35;
group 6), a sense strand set forth in SEQ ID NO: 6 and an antisense strand set forth in SEQ ID NO: 36;
group 7), a sense strand set forth in SEQ ID NO: 7 and an antisense strand set forth in SEQ ID NO: 37;
group 8), a sense strand set forth in SEQ ID NO: 8 and an antisense strand set forth in SEQ ID NO: 38;
group 9), a sense strand set forth in SEQ ID NO: 9 and an antisense strand set forth in SEQ ID NO: 39; and
group 10), a sense strand set forth in SEQ ID NO: 10 and an antisense strand set forth in SEQ ID NO: 40.

In some embodiments, at least one nucleotide in the sense strand and/or the antisense strand is a modified nucleotide.

In some embodiments, all of the nucleotides are modified nucleotides.

In some embodiments, three contiguous nucleotides in the sense strand of the siRNA are 2'-fluoro-modified nucleotides.

In some embodiments, in the sense strand of the siRNA, in the direction from the 5' end to the 3' end, nucleotides at positions 7, 8, and 9 of the sense strand are 2'-fluoro-modified nucleotides.

In some embodiments, in the sense strand of the siRNA, in the direction from the 5' end to the 3' end, nucleotides at positions 7, 8, and 9 of the sense strand are 2'-fluoro-modified nucleotides, and nucleotides at the remaining positions of the sense strand are all 2'-methoxy-modified nucleotides.

In some embodiments, in the direction from the 5' end to the 3' end, nucleotides at positions 2, 6, 12, 14, and 16 of the antisense strand are each independently a 2'-fluoro-modified nucleotide. In some embodiments, a nucleotide at

position 2, 4, 6, 10, 12, 14, 16, or 18 of the antisense strand is each independently a 2'-fluoro-modified nucleotide. In some embodiments, nucleotides at the remaining positions of the antisense strand are all 2'-methoxy-modified nucleotides.

In some embodiments, at least one phosphodiester group in the sense strand and/or the antisense strand is a phosphodiester group with a modification group. The modification group endows the siRNA with increased stability in a biological sample or environment. In some embodiments, the phosphodiester group with a modification group is a phosphorothioate diester group.

In some embodiments, the phosphodiester group with a modification group is present at at least one of the following positions selected from the group consisting of:

a position between the 1st and 2nd nucleotides at the 5' end of the sense strand;
a position between the 2nd and 3rd nucleotides at the 5' end of the sense strand;
a position between the 1st and 2nd nucleotides at the 5' end of the antisense strand;
a position between the 2nd and 3rd nucleotides at the 5' end of the antisense strand;
a position between the 1st and 2nd nucleotides at the 3' end of the antisense strand; and
a position between the 2nd and 3rd nucleotides at the 3' end of the antisense strand.

In some embodiments, the sense strand and/or the antisense strand comprise a plurality of phosphodiester groups with a modification group.

In some embodiments, the sense strand and/or the antisense strand comprise a plurality of phosphodiester groups with a modification group, and the phosphodiester groups with a modification group are present:

between the 1st and 2nd nucleotides at the 5' end of the sense strand; and
between the 2nd and 3rd nucleotides at the 5' end of the sense strand; and
between the 1st and 2nd nucleotides at the 5' end of the antisense strand; and
between the 2nd and 3rd nucleotides at the 5' end of the antisense strand; and
between the 1st and 2nd nucleotides at the 3' end of the antisense strand; and
a position between the 2nd and 3rd nucleotides at the 3' end of the antisense strand.

In some embodiments, the sense strand is selected from the group consisting of or comprises a nucleotide sequence represented by the following formula:
5'-NmsNmsNmNmNmNmNfNfNfNmNmNmNmNmNmNmNmNmNm-3',

In some embodiments, the antisense strand is selected from the group consisting of or comprises a nucleotide sequence represented by the following formula:

5'-Nm'sNf'sNm'Nm'Nm'Nf'Nm'Nm'Nm'Nm'Nm'NfNm'Nf'Nm'Nf'Nm'Nm'Nm'sNm'sNm'-3'; or
5'-Nm'sNf'sNm'Nf'Nm'NfNm'Nm'Nm'Nf'Nm'Nf'Nm'Nf'Nm'NfNm'Nf'Nm'sNm'sNm'-3'.

In the sense strand and the antisense strand described above, Nm and Nm' represent any 2'-methoxy-modified nucleoside, such as 2'-methoxy-modified C, G, U, A, or T; Nf and Nf' represent any 2'-fluoro-modified nucleoside, such as 2'-fluoro-modified C, G, U, A, or T;
the lowercase letter s indicates that two adjacent nucleotides flanking the letter s are linked by a phosphorothioate diester group. Unless otherwise specified, two adjacent nucleosides are linked by a phosphodiester group.

In some embodiments, at least one nucleoside at positions 2 to 8 (e.g., position 2, position 3, position 4, position 5, position 6, position 7, or position 8) of the 5' region of the antisense strand comprises a chemical modification represented by formula (I) or a pharmaceutically acceptable salt thereof, and the chemical modification represented by formula (I) is selected from the group consisting of:

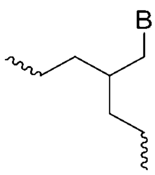 , 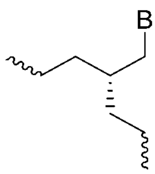 , and 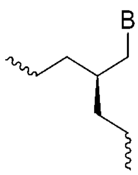 ,

wherein: B represents a base corresponding to positions 2-8 at the 5' end of the antisense strand, and the nucleoside comprising the chemical modification represented by formula (I) or the pharmaceutically acceptable salt thereof is linked to a nucleoside adjacent thereto by a phosphodiester group or a phosphodiester group with a modification group.

In some embodiments, the sense strand is selected from the group consisting of or comprises: the nucleotide

sequence set forth in any one of SEQ ID NO: 11 to SEQ ID NO: 27 and SEQ ID NO: 243 to SEQ ID NO: 333.

In some embodiments, the antisense strand is selected from the group consisting of or comprises: the nucleotide sequence set forth in any one of SEQ ID NO: 41 to SEQ NO: 57 and SEQ ID NO: 334 to SEQ ID NO: 424.

In more specific embodiments, the present disclosure provides an siRNA, which comprises or is selected from any one of the following groups:

the sense strand comprising a polynucleotide set forth in SEQ ID NO: 11, and the antisense strand comprising a polynucleotide set forth in SEQ ID NO: 41;
the sense strand comprising a polynucleotide set forth in SEQ ID NO: 12, and the antisense strand comprising a polynucleotide set forth in SEQ ID NO: 42;
the sense strand comprising a polynucleotide set forth in SEQ ID NO: 13, and the antisense strand comprising a polynucleotide set forth in SEQ ID NO: 43;
the sense strand comprising a polynucleotide set forth in SEQ ID NO: 14, and the antisense strand comprising a polynucleotide set forth in SEQ ID NO: 44;
the sense strand comprising a polynucleotide set forth in SEQ ID NO: 15, and the antisense strand comprising a polynucleotide set forth in SEQ ID NO: 45;
the sense strand comprising a polynucleotide set forth in SEQ ID NO: 16, and the antisense strand comprising a polynucleotide set forth in SEQ ID NO: 46;
the sense strand comprising a polynucleotide set forth in SEQ ID NO: 17, and the antisense strand comprising a polynucleotide set forth in SEQ ID NO: 47;
the sense strand comprising a polynucleotide set forth in SEQ ID NO: 18, and the antisense strand comprising a polynucleotide set forth in SEQ ID NO: 48;
the sense strand comprising a polynucleotide set forth in SEQ ID NO 19, and the antisense strand comprising a polynucleotide set forth in SEQ ID NO: 49;
the sense strand comprising a polynucleotide set forth in SEQ ID NO: 20, and the antisense strand comprising a polynucleotide set forth in SEQ ID NO: 50;
the sense strand comprising a polynucleotide set forth in SEQ ID NO 21, and the antisense strand comprising a polynucleotide set forth in SEQ ID NO: 51;
the sense strand comprising a polynucleotide set forth in SEQ ID NO: 22, and the antisense strand comprising a polynucleotide set forth in SEQ ID NO: 52;
the sense strand comprising a polynucleotide set forth in SEQ ID NO: 23, and the antisense strand comprising a polynucleotide set forth in SEQ NO: 53;
the sense strand comprising a polynucleotide set forth in SEQ ID NO 24, and the antisense strand comprising a polynucleotide set forth in SEQ ID NO: 54;
the sense strand comprising a polynucleotide set forth in SEQ ID NO: 25, and the antisense strand comprising a polynucleotide set forth in SEQ ID NO: 55;
the sense strand comprising a polynucleotide set forth in SEQ ID NO: 26, and the antisense strand comprising a polynucleotide set forth in SEQ ID NO: 56;
the sense strand comprising a polynucleotide set forth in SEQ ID NO 27, and the antisense strand comprising a polynucleotide set forth in SEQ ID NO: 57.

The present disclosure also provides an siRNA conjugate, which comprises any one of the siRNAs described above and a targeting ligand linked to the siRNA.

"siRNA conjugate" refers to a compound formed by linking one or more chemical moieties to an siRNA.

In some embodiments, the siRNA and the targeting ligand are linked covalently or non-covalently.

In some embodiments, the targeting ligand is linked to the antisense strand of the siRNA. In some embodiments, the targeting ligand is linked to the 5' end of the antisense strand of the siRNA. In some embodiments, the targeting ligand is linked to the 3' end of the antisense strand of the siRNA.

In some embodiments, the targeting ligand is linked to the sense strand of the siRNA. In some embodiments, the targeting ligand is linked to the 5' end of the sense strand of the siRNA. In some embodiments, the targeting ligand is linked to the 3' end of the sense strand of the siRNA.

In some embodiments, the targeting ligand is linked to an end of the siRNA by a phosphodiester group, a phosphorothioate diester group, or a phosphonic acid group; in some embodiments, the targeting ligand is linked to an end of the siRNA by a phosphodiester group.

In some embodiments, the targeting ligand is indirectly linked to an end of the siRNA by a phosphodiester group, a phosphorothioate diester group, or a phosphonic acid group. In some embodiments, the targeting ligand is indirectly linked to an end of the siRNA by a phosphodiester group. In some embodiments, the targeting ligand is directly linked to an end of the siRNA by a phosphodiester group, a phosphorothioate diester group, or a phosphonic acid group. In some

embodiments, the targeting ligand is directly linked to an end of the siRNA by a phosphodiester group.

In some embodiments, the targeting ligand is directly linked to the 3' end of the sense strand of the siRNA by a phosphodiester group or a phosphorothioate diester group. In some embodiments, the targeting ligand is directly linked to the 3' end of the sense strand of the siRNA by a phosphodiester group.

In some embodiments, one or more said targeting ligands are linked to the sense strand of the siRNA.

In some embodiments, the targeting ligand has affinity for a cellular receptor expressed on an epithelial cell.

In some embodiments, the targeting ligand includes an integrin-targeting ligand.

In some embodiments, the targeting ligand includes an αvβ6 integrin-targeting ligand. The αvβ6 integrin-targeting ligand may be any targeting ligand capable of binding to αvβ6 integrin. In some embodiments, the targeting ligand has the following structure:

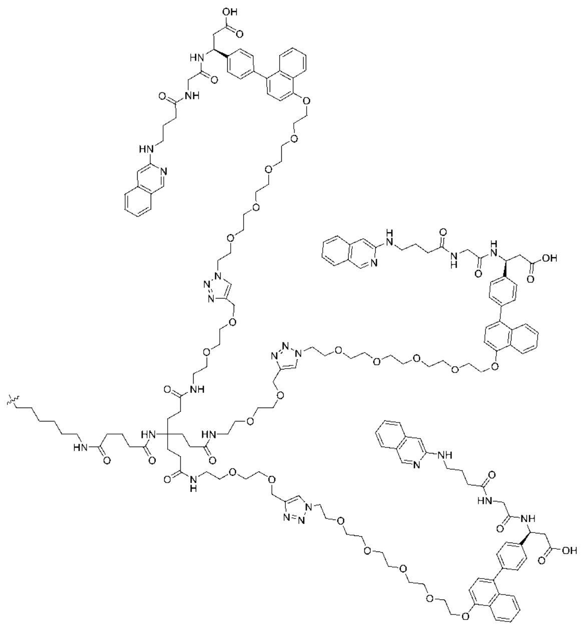

In some embodiments, the targeting ligand targets the liver.

In some embodiments, the targeting ligand binds to an asialoglycoprotein receptor (ASGPR).

In some embodiments, the targeting ligand comprises a galactose cluster or a galactose derivative cluster, wherein the galactose derivative is selected from the group consisting of N-acetyl-galactosamine, N-trifluoroacetylgalactosamine, N-propionylgalactosamine, N-n-butyrylgalactosamine, and N-isobutyrylgalactosamine.

Targeting ligands that bind to asialoglycoprotein receptors (ASGPRs) are known to be particularly used for directing the delivery of oligomeric compounds to the liver. Asialoglycoprotein receptors are extensively expressed on liver cells (hepatocytes). The targeting moieties of cellular receptors targeting ASGPRs include galactose and galactose derivatives. Specifically, clusters of galactose derivatives, including clusters consisting of 2, 3, 4, or more than 4 N-acetyl-galactosamine (GalNAc or NAG) molecules, can promote the uptake of certain compounds in hepatocytes. The GalNAc cluster coupled to the oligomeric compound is used for directing the composition to the liver, where the N-acetyl-galactosamine saccharide can bind to the asialoglycoprotein receptors on the liver cell surface. It is believed that the binding to the asialoglycoprotein receptors will initiate receptor-mediated endocytosis, thereby promoting entry of the compound into the interior of the cell.

In some embodiments, the targeting ligand provided by the present disclosure is a compound shown below or a pharmaceutically acceptable salt thereof:

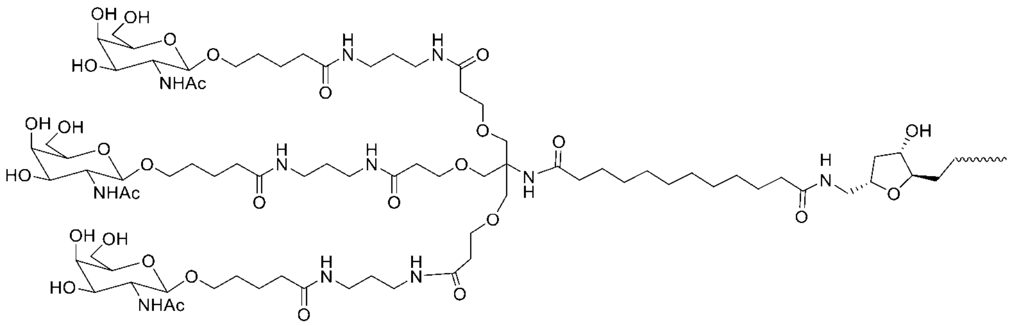

,

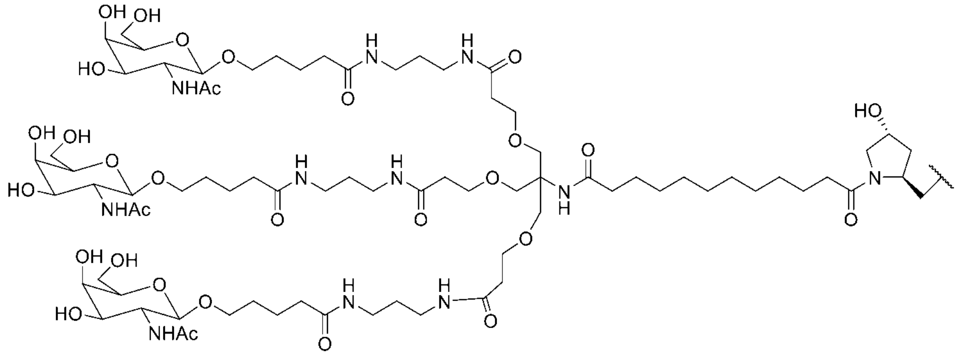

.

In some embodiments, the N-acetyl-galactosamine moiety in the above targeting ligands may be replaced with N-trifluoroacetylgalactosamine, N-propionylgalactosamine, N-n-butyrylgalactosamine, or N-isobutyrylgalactosamine.

In some embodiments, in the siRNA conjugate described in the present disclosure, the sense strand comprises the nucleotide sequence set forth in any one of SEQ ID NO: 28 to SEQ ID NO: 30; and/or the antisense strand comprises the nucleotide sequence set forth in any one of SEQ ID NO: 58 to SEQ ID NO: 60.

In some specific embodiments, the siRNA conjugate of the present disclosure is selected from any one of the following groups:

the sense strand comprising a polynucleotide set forth in SEQ ID NO: 28, and the antisense strand comprising a polynucleotide set forth in SEQ ID NO: 58;
the sense strand comprising a polynucleotide set forth in SEQ ID NO: 29, and the antisense strand comprising a polynucleotide set forth in SEQ ID NO: 59;
the sense strand comprising a polynucleotide set forth in SEQ ID NO: 30, and the antisense strand comprising a polynucleotide set forth in SEQ ID NO: 60.

In another aspect, the present disclosure provides a pharmaceutical composition, which comprises the siRNA or the siRNA conjugate described in the present disclosure, and one or more pharmaceutically acceptable excipients, such as vehicles, carriers, diluents, and/or delivery polymers. The term "pharmaceutical composition" refers to a mixture containing one or more of the compounds or the physiologically and pharmaceutically acceptable salts or pro-drugs thereof described herein, and other chemical components, as well as other components such as physiologically and pharmaceutically acceptable carriers and excipients. The pharmaceutical composition is intended to promote administration to an organism and facilitate the absorption of the active ingredient so that it can exert its biological activity.

Various delivery systems are known and can be used for the siRNA or the siRNA conjugate of the present disclosure, e.g., encapsulation in liposomes, microparticles, microcapsules, recombinant cells capable of expressing the siRNA or the siRNA conjugate, receptor-mediated endocytosis, and construction of a nucleic acid as part of retroviral or other vectors.

"Pharmaceutically acceptable excipient" includes, but is not limited to, any auxiliary, carrier, glidant, sweetener, diluent, preservative, dye/colorant, flavoring agent, surfactant, wetting agent, dispersant, suspending agent, stabilizer, isotonic agent, solvent, or emulsifier that has been approved by the U.S. Food and Drug Administration (FDA) as acceptable for use in humans or livestock animals.

In another aspect, the present disclosure provides use of the siRNA, the siRNA conjugate, or the pharmaceutical composition described above in the manufacture of a medicament for treating a disease in a subject.

In another aspect, the present disclosure provides a method for treating a disease in a subject, which comprises administering to the subject the siRNA, the siRNA conjugate, or the pharmaceutical composition described above.

In another aspect, the present disclosure provides a method for inhibiting mRNA expression in a subject, and the method comprises administering to the subject the siRNA, the siRNA conjugate, or the pharmaceutical composition described above.

In another aspect, the present disclosure provides a method for delivering an expression-inhibiting oligomeric compound *in vivo,* and the method comprises administering to a subject the siRNA, the siRNA conjugate, or the pharmaceutical composition described above.

In some embodiments, the oligomeric compound is delivered to the liver.

In some embodiments, the oligomeric compound is delivered extrahepatically.

In some embodiments, the oligomeric compound is delivered to the lung.

The siRNA, the siRNA conjugate, or the pharmaceutical composition, and the method disclosed herein can reduce the level of a target mRNA in a cell, a cell population, a tissue, or a subject, and inhibition of the expression of the target mRNA in the subject is achieved by administering to the subject the siRNA, the siRNA conjugate, or the pharmaceutical composition described in the present disclosure. In some embodiments, an effective amount or an effective dose of the siRNA, the siRNA conjugate, or the pharmaceutical composition described in the present disclosure is administered to a subject.

In some embodiments, it has been previously identified that the subject has pathological up-regulation of the target gene in the targeted cell or tissue.

The subject described in the present disclosure refers to a subject having (or suspected to have or susceptible to) a disease or disorder that would benefit from reduction or inhibition of target mRNA expression.

Delivery can be accomplished by topical administration (e.g., direct injection, implantation, or topical application), systemic administration, or through subcutaneous, intravenous, intraperitoneal, or parenteral routes, including intracranial (e.g., intraventricular, intraparenchymal, and intrathecal), intramuscular, transdermal, airway (aerosol), nasal, oral, rectal, or topical (including buccal and sublingual) administration. In an optional embodiment, the pharmaceutical composition provided by the present disclosure may be administered by injection, e.g., intravenous, intramuscular, intradermal, subcutaneous, intraduodenal, or intraperitoneal injection.

In an optional embodiment, after the targeting ligand and siRNA are linked to form a conjugate, the conjugate can be packaged in a kit.

In another aspect, the present disclosure also provides a pharmaceutical composition, which comprises the siRNA or the siRNA conjugate of the present disclosure.

In some embodiments, the pharmaceutical composition may further comprise a pharmaceutically acceptable auxiliary material and/or adjuvant, and the auxiliary material may be one or more of the various formulations or compounds conventionally used in the art. For example, the pharmaceutically acceptable auxiliary material may include at least one of a pH buffer, a protective agent, and an osmotic pressure regulator.

In some embodiments, a unit dose of the pharmaceutical composition is 0.001 mg to 1000 mg.

In some embodiments, the pharmaceutical composition comprises 0.01% to 99.99% of the aforementioned compound or a pharmaceutically acceptable salt thereof or an isotopically substituted form thereof based on the total weight of the composition. In some embodiments, the pharmaceutical composition comprises 0.1% to 99.9% of the aforementioned compound or the pharmaceutically acceptable salt thereof or the isotopically substituted form thereof. In some embodiments, the pharmaceutical composition comprises 0.5% to 99.5% of the aforementioned compound or the pharmaceutically acceptable salt thereof or the isotopically substituted form thereof. In some embodiments, the pharmaceutical composition comprises 1% to 99% of the aforementioned compound or the pharmaceutically acceptable salt thereof or the isotopically substituted form thereof. In some embodiments, the pharmaceutical composition comprises 2% to 98% of the aforementioned compound or the pharmaceutically acceptable salt thereof or the isotopically substituted form thereof.

The pharmaceutical composition may conveniently be presented in a unit dosage form. In general, the pharmaceutical composition may be formulated into any suitable dosage form, such as, but not limited to, injections, tablets, capsules, gels, and the like. The pharmaceutical composition includes, but is not limited to, a solution, an emulsion, and a liposome-containing formulation.

In some embodiments, the pharmaceutical composition comprises 0.01% to 99.99% of a pharmaceutically acceptable excipient based on the total weight of the composition. In some embodiments, the pharmaceutical composition comprises 0.1% to 99.9% of the pharmaceutically acceptable excipient. In some embodiments, the pharmaceutical composition comprises 0.5% to 99.5% of the pharmaceutically acceptable excipient. In some embodiments, the pharmaceutical composition comprises 1% to 99% of the pharmaceutically acceptable excipient. In some embodiments, the pharmaceutical composition comprises 2% to 98% of the pharmaceutically acceptable excipient.

In some embodiments, when the siRNA, the siRNA conjugate, or the pharmaceutical composition described above is in contact with a target gene-expressing cell, the siRNA conjugate or the pharmaceutical composition described above inhibits the target gene expression by at least 5%, at least 10%, at least 15%, at least 20%, at least 25%, at least 30%, at least 35%, at least 40%, at least 45%, at least 50%, at least 55%, at least 60%, at least 65%, at least 70%, at least

75%, at least 80%, at least 85%, at least 90%, at least 91%, at least 92%, at least 93%, at least 94%, at least 95%, at least 96%, at least 97%, at least 98%, or at least 99%, as measured by, for example, psiCHECK activity screening and luciferase reporter gene assay, other methods such as PCR or branched DNA (bDNA)-based methods, or protein-based methods such as immunofluorescence assay, e.g., western blot or flow cytometry.

In some embodiments, when the siRNA, the siRNA conjugate, or the pharmaceutical composition described above is in contact with a target gene-expressing cell, the siRNA conjugate or the pharmaceutical composition described above results in a remaining percentage of target gene mRNA expression of no more than 99%, no more than 95%, no more than 90%, no more than 85%, no more than 80%, no more than 75%, no more than 70%, no more than 65%, no more than 60%, no more than 55%, no more than 50%, no more than 45%, no more than 40%, no more than 35%, no more than 30%, no more than 25%, no more than 20%, no more than 15%, or no more than 10%, as measured by, for example, psiCHECK activity screening and luciferase reporter gene assay, other methods such as PCR or branched DNA (bDNA)-based methods, or protein-based methods such as immunofluorescence assay, e.g., western blot or flow cytometry.

In some embodiments, when the siRNA, the siRNA conjugate, or the pharmaceutical composition described above is in contact with a target gene-expressing cell, the siRNA conjugate reduces off-target activity by at least 20%, at least 25%, at least 30%, at least 35%, at least 40%, at least 45%, at least 50%, at least 55%, at least 60%, at least 65%, at least 70%, or at least 75%, while maintaining on-target activity, as measured by, for example, psiCHECK activity screening and luciferase reporter gene assay, other methods such as PCR or branched DNA (bDNA)-based methods, or protein-based methods such as immunofluorescence assay, e.g., western blot or flow cytometry.

In some embodiments, when the siRNA, the siRNA conjugate, or the pharmaceutical composition described above is in contact with a target gene-expressing cell, the siRNA conjugate reduces off-target activity by at least 20%, at least 25%, at least 30%, at least 35%, at least 40%, at least 45%, at least 50%, at least 55%, at least 60%, at least 65%, at least 70%, or at least 75%, while reducing on-target activity by at most 20%, at most 19%, at most 15%, at most 10%, at most 5%, or more than 1%, as measured by, for example, psiCHECK activity screening and luciferase reporter gene assay, other methods such as PCR or branched DNA (bDNA)-based methods, or protein-based methods such as immunofluorescence assay, e.g., western blot or flow cytometry.

In some embodiments, when the siRNA, the siRNA conjugate, or the pharmaceutical composition described above is in contact with a target gene-expressing cell, the siRNA conjugate reduces off-target activity by at least 20%, at least 25%, at least 30%, at least 35%, at least 40%, at least 45%, at least 50%, at least 55%, at least 60%, at least 65%, at least 70%, or at least 75%, while increasing on-target activity by at least 1%, at least 5%, at least 10%, at least 15%, at least 20%, at least 25%, at least 30%, at least 35%, at least 40%, at least 45%, at least 50%, at least 55%, at least 60%, at least 65%, at least 70%, at least 75%, or at least 80%, as measured by, for example, psiCHECK activity screening and luciferase reporter gene assay, other methods such as PCR or branched DNA (bDNA)-based methods, or protein-based methods such as immunofluorescence assay, e.g., western blot or flow cytometry.

The present disclosure also provides a cell, which comprises the siRNA and/or the siRNA conjugate of the present disclosure.

The present disclosure also provides a kit, which comprises the siRNA and/or the siRNA conjugate and/or the pharmaceutical composition of the present disclosure. In some embodiments, the kit comprises one or more containers, and the one or more containers comprise the siRNA and/or the siRNA conjugate and/or the pharmaceutical composition of the present disclosure.

The present disclosure also provides a method for silencing a target gene or mRNA of the target gene in a cell, and the method comprises a step of introducing into the cell the siRNA, the siRNA conjugate, and/or the pharmaceutical composition according to the present disclosure.

The present disclosure also provides a method for silencing a target gene or mRNA of the target gene in a cell *in vivo* or *in vitro,* and the method comprises a step of introducing into the cell the siRNA, the siRNA conjugate, and/or the pharmaceutical composition according to the present disclosure.

The present disclosure also provides a method for reducing the expression of a target gene or mRNA of the target gene, and the method comprises administering to a subject in need thereof the siRNA and/or the siRNA conjugate and/or the pharmaceutical composition of the present disclosure. In some embodiments, the method comprises administering to a subject in need thereof an effective amount or an effective dose of the siRNA and/or the siRNA conjugate of the present disclosure.

In some embodiments, administration is performed through administration modes including intramuscular, intrabronchial, intrapleural, intraperitoneal, intraarterial, lymphatic, intravenous, subcutaneous, cerebrospinal, or combinations thereof.

In some embodiments, the effective amount or the effective dose of the siRNA, the siRNA conjugate, and/or the pharmaceutical composition is about 0.001 mg/kg body weight to about 200 mg/kg body weight, about 0.01 mg/kg body weight to about 100 mg/kg body weight, or about 0.5 mg/kg body weight to about 50 mg/kg body weight.

In some embodiments, the target gene is matrix metalloproteinase 7 (MMP7).

The present disclosure provides the aforementioned siRNA and/or pharmaceutical composition and/or siRNA conjugate for use in treating and/or preventing a disease associated with matrix metalloproteinase 7 (MMP7) gene expression in a subject. In some embodiments, the disease is selected from the group consisting of idiopathic pulmonary fibrosis (IPF), asthma, other types of fibrosis, chronic inflammation, interstitial lung disease (ILD), SARS-COV-2 or other types of infectious diseases, acute respiratory distress syndrome (ARDS) or other types of acute lung injury, pulmonary hypertension, cancer, renal fibrosis, and liver fibrosis.

The present disclosure provides a method for treating and/or preventing a disease associated with MMP7 gene expression in a subject, which comprises administering to the subject the siRNA and/or the siRNA conjugate and/or the pharmaceutical composition described in the present disclosure. In some embodiments, the method comprises administering to the subject an effective amount or an effective dose of the siRNA and/or the siRNA conjugate and/or the pharmaceutical composition described in the present disclosure. In some embodiments, the disease is selected from the group consisting of idiopathic pulmonary fibrosis (IPF), asthma, other types of fibrosis, chronic inflammation, interstitial lung disease (ILD), SARS-COV-2 or other types of infectious diseases, acute respiratory distress syndrome (ARDS) or other types of acute lung injury, pulmonary hypertension, cancer, renal fibrosis, and liver fibrosis.

The present disclosure provides use of the aforementioned siRNA and/or pharmaceutical composition and/or siRNA conjugate in the manufacture of a medicament for treating and/or preventing a disease associated with MMP7 gene expression. In some embodiments, the disease is selected from the group consisting of idiopathic pulmonary fibrosis (IPF), asthma, other types of fibrosis, chronic inflammation, interstitial lung disease (ILD), SARS-COV-2 or other types of infectious diseases, acute respiratory distress syndrome (ARDS) or other types of acute lung injury, pulmonary hypertension, cancer, renal fibrosis, and liver fibrosis.

The present disclosure provides use of the aforementioned siRNA and/or pharmaceutical composition and/or siRNA conjugate in the manufacture of a medicament for inhibiting the expression of MMP7.

The present disclosure provides a method for inhibiting the expression of MMP7, which comprises administering to a subject the aforementioned siRNA and/or pharmaceutical composition and/or siRNA conjugate of the present disclosure. In some embodiments, the method comprises administering to the subject an effective amount or an effective dose of the siRNA and/or the pharmaceutical composition and/or the siRNA conjugate of the present disclosure.

The present disclosure provides the aforementioned siRNA and/or pharmaceutical composition and/or siRNA conjugate for use in treating and/or preventing a disease. In some embodiments, the disease is selected from the group consisting of idiopathic pulmonary fibrosis (IPF), asthma, other types of fibrosis, chronic inflammation, interstitial lung disease (ILD), SARS-COV-2 or other types of infectious diseases, acute respiratory distress syndrome (ARDS) or other types of acute lung injury, pulmonary hypertension, cancer, renal fibrosis, and liver fibrosis.

The present disclosure provides a method for treating and/or preventing a disease, which comprises administering to a subject the aforementioned siRNA and/or pharmaceutical composition and/or siRNA conjugate. In some embodiments, the method comprises administering to the subject an effective amount or an effective dose of the siRNA and/or the pharmaceutical composition and/or the conjugate of the present disclosure. In some embodiments, the disease is selected from the group consisting of idiopathic pulmonary fibrosis (IPF), asthma, other types of fibrosis, chronic inflammation, interstitial lung disease (ILD), SARS-COV-2 or other types of infectious diseases, acute respiratory distress syndrome (ARDS) or other types of acute lung injury, pulmonary hypertension, cancer, renal fibrosis, and liver fibrosis.

The present disclosure provides use of the aforementioned siRNA and/or pharmaceutical composition and/or siRNA conjugate in the manufacture of a medicament for treating and/or preventing a disease. In some embodiments, the disease is selected from the group consisting of idiopathic pulmonary fibrosis (IPF), asthma, other types of fibrosis, chronic inflammation, interstitial lung disease (ILD), SARS-COV-2 or other types of infectious diseases, acute respiratory distress syndrome (ARDS) or other types of acute lung injury, pulmonary hypertension, cancer, renal fibrosis, and liver fibrosis.

The present disclosure provides a method for delivering an siRNA that inhibits the expression and/or replication of MMP7 *in vivo,* and the method comprises administering to a subject the aforementioned siRNA and/or pharmaceutical composition and/or siRNA conjugate.

In some embodiments, the siRNA is delivered to the liver.

In some embodiments, the siRNA is delivered extrahepatically.

In some embodiments, the siRNA is delivered to the lung.

The present disclosure also provides a method for preparing an siRNA or an siRNA conjugate, which comprises synthesizing the siRNA or the siRNA conjugate described in the present disclosure.

The present disclosure also provides an siRNA or an siRNA conjugate, wherein one or more bases U, e.g., 1, 2, 3, 3, 5, 6, 7, 8, 9, or 10 bases U, of any one of the siRNAs or the siRNA conjugates of the present disclosure are replaced with bases T.

The pharmaceutically acceptable salts of the compounds described in the present disclosure are selected from the group consisting of inorganic salts and organic salts. The compounds described in the present disclosure can react with acidic or basic substances to form corresponding salts.

In another aspect, where the configuration is not specified, the compounds of the present disclosure may be

present in specific geometric or stereoisomeric forms. The present disclosure contemplates all such compounds, including cis and trans isomers, (-)- and (+)-enantiomers, (R)- and (S)-enantiomers, diastereomers, (D)-isomers, (L)-isomers, and racemic mixtures and other mixtures thereof, such as enantiomerically or diastereomerically enriched mixtures, all of which are within the scope of the present disclosure. Additional asymmetric carbon atoms may be present in substituents such as an alkyl group. All such isomers and mixtures thereof are included within the scope of the present disclosure.

In addition, where the configuration is not specified, the compounds and intermediates of the present disclosure may also be present in different tautomeric forms, and all such forms are included within the scope of the present disclosure. The term "tautomer" or "tautomeric form" refers to structural isomers of different energies that can interconvert via a low-energy barrier.

All compounds in the present disclosure can be drawn as form A or form B. All tautomeric forms are within the scope of the present disclosure. The names of the compounds do not exclude any tautomers.

The compounds of the present disclosure may be asymmetric, for example, having one or more stereoisomers. Unless otherwise specified, all stereoisomers are included, for example, enantiomers and diastereomers. The compounds of the present disclosure containing asymmetric carbon atoms may be separated in an optically active pure form or in a racemic form. The optically active pure form may be isolated from a racemic mixture or synthesized using chiral starting materials or chiral reagents.

Optically active (R)- and (S)-enantiomers and D- and L-isomers can be prepared by chiral synthesis, chiral reagents, or other conventional techniques. If one enantiomer of a certain compound of the present disclosure is desired, it may be prepared by asymmetric synthesis or derivatization with a chiral auxiliary, wherein the resulting mixture of diastereomers is separated and the auxiliary group is cleaved to provide the pure desired enantiomer. Alternatively, when the molecule contains a basic functional group (e.g., amino) or an acidic functional group (e.g., carboxyl), salts of diastereomers are formed with an appropriate optically active acid or base, diastereomeric resolution is then performed by conventional methods well-known in the art, and pure enantiomers are then recovered. In addition, the separation of enantiomers and diastereomers is generally accomplished by chromatography using a chiral stationary phase, optionally in combination with chemical derivatization (e.g., carbamate formation from amines).

The present disclosure also includes some isotopically labeled compounds of the present disclosure that are identical to those recited herein but have one or more atoms replaced with an atom having an atomic mass or mass number different from the atomic mass or mass number usually found in nature. Examples of isotopes that can be incorporated into the compounds of the present disclosure include isotopes of hydrogen, carbon, nitrogen, oxygen, phosphorus, sulfur, fluorine, iodine, and chlorine, such as $^{2}H$, $^{3}H$, $^{11}C$, $^{13}C$, $^{14}C$, $^{13}N$, $^{15}N$, $^{15}O$, $^{17}O$, $^{18}O$, $^{31}P$, $^{32}P$, $^{35}S$, $^{18}F$, $^{123}I$, $^{125}I$, and $^{36}Cl$.

Unless otherwise specified, when a position is specifically designated as deuterium (D), the position shall be understood to be deuterium having an abundance that is at least 1000 times greater than the natural abundance of deuterium (which is 0.015%) (i.e., at least 10% deuterium incorporation). The compounds of examples comprise deuterium having an abundance that is greater than at least 1000 times the natural abundance, at least 2000 times the natural abundance, at least 3000 times the natural abundance, at least 4000 times the natural abundance, at least 5000 times the natural abundance, at least 6000 times the natural abundance, or higher times the natural abundance. The present disclosure further comprises various deuterated forms of the compounds. Each available hydrogen atom linked to a carbon atom may be independently replaced with a deuterium atom. Those skilled in the art can synthesize the compounds in deuterated form by reference to the relevant literature. Commercially available deuterated starting materials can be used in preparing the deuterated compounds, or they can be synthesized using conventional techniques with deuterated reagents, including but not limited to deuterated borane, tri-deuterated borane in tetrahydrofuran, deuterated lithium aluminum hydride, deuterated iodoethane, deuterated iodomethane, and the like.

Where the configurations are not specified, in the chemical structures of the compounds of the present disclosure, a bond "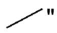" indicates an unspecified configuration; that is, if chiral isomers exist in the chemical structures, the bond "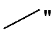" may be "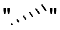" or "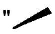", or includes both the configurations "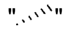" and "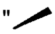". Although all of the above structural formulas are drawn as certain isomeric forms for the sake of simplicity, the present disclosure may include all isomers, such as tautomers, rotamers, geometric isomers, diastereomers, racemates, and enantiomers. In the chemical structures of the compounds of the present disclosure, a bond "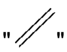" does not specify a configuration; that is, the configuration for the bond "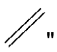" can be an E configuration or a Z configuration, or includes both the E configuration and the Z configuration.

WO2022028462A1, WO2023274395A1, and WO2023208023A1 are incorporated in the present disclosure by reference in their entirety.

**Terms and Definitions**

In order to facilitate the understanding of the present disclosure, some technical and scientific terms are specifically defined below. Unless otherwise specifically defined herein, all other technical and scientific terms used herein have the meanings generally understood by those of ordinary skill in the art to which the present disclosure pertains. Unless otherwise specified, in the context of the present disclosure, the terms "matrix metalloproteinase 7" and "MMP7" are used interchangeably in the present disclosure. MMP7 includes, but is not limited to, human MMP7, cynomolgus monkey MMP7, mouse MMP7, and rat MMP7, the amino acids and complete coding sequences and mRNA sequences of which are readily accessible using publicly available databases, e.g., GenBank, UniProt, OMIM, and the Macaca genome project website.

The term "MMP7" also refers to naturally occurring DNA sequence variations of the MMP7 gene, such as a single-nucleotide polymorphism (SNP) in the MMP7 gene. Exemplary SNPs may be found in the dbSNP database.

The term "target sequence" refers to a contiguous portion of the nucleotide sequence of an mRNA molecule formed during the transcription of MMP7, including mRNA that is a product of RNA processing of a primary transcription product. The targeted portion in the target sequence should be long enough to serve as a substrate for iRNA-directed cleavage.

In one embodiment, the target sequence is within the protein-coding region of MMP7. In the context of the present disclosure, "target sequence" and "target mRNA" have the same meaning and are used interchangeably.

Targeting groups described herein may include cellular receptor ligands, such as integrin-targeting ligands. Integrins are a family of transmembrane receptors that promote cell-extracellular matrix (ECM) adhesion. Among them, integrin alpha-v-beta-6 ($\alpha v\beta 6$) is an epithelium-specific integrin. $\alpha v\beta 6$ is known as a receptor for ECM proteins and TGF-$\beta$ latency-associated peptide (LAP), and is expressed in various cells and tissues. Moreover, integrin $\alpha v\beta 6$ is highly up-regulated in injured lung epithelial cells.

The MMP7 siRNAs described herein may be linked to an integrin-targeting ligand having affinity for integrin $\alpha v\beta 6$. As referred to herein, "$\alpha v\beta 6$ integrin-targeting ligand" is a compound having affinity for integrin $\alpha v\beta 6$, which can be used as a ligand to facilitate the targeting and delivery of the siRNA to which it is attached to a desired cell and/or tissue (i.e., a cell expressing integrin $\alpha v\beta 6$).

As used herein, in the context of RNA-mediated gene silencing, the sense strand (also referred to as SS or SS strand) refers to a strand that comprises a sequence that is identical or substantially identical to a target mRNA sequence; the antisense strand (also referred to as AS or AS strand) refers to a strand having a sequence complementary to a target mRNA sequence.

In the context describing the siRNA sense strand described herein, the term "a sequence differing from any one of the nucleotide sequences of SEQ ID NO: 1 to SEQ ID NO: 10 by no more than 3 nucleotides and comprises at least 15 contiguous nucleotides" is intended to mean that the siRNA sense strand described herein comprises at least 15 contiguous nucleotides of any one of the sense strands of SEQ ID NO: 1 to SEQ ID NO: 10 or a sequence differing from at least 15 contiguous nucleotides of any one of the sense strands of SEQ ID NO: 1 to SEQ ID NO: 10 by no more than 3 nucleotides (optionally, by no more than 2 nucleotides; optionally, by 1 nucleotide). Optionally, the siRNA sense strand described herein comprises at least 16 contiguous nucleotides of any one of the sense strands of SEQ ID NO: 1 to SEQ ID NO: 10 or a sequence differing from at least 16 contiguous nucleotides of any one of the sense strands of SEQ ID NO: 1 to SEQ ID NO: 10 by no more than 3 nucleotides (optionally, by no more than 2 nucleotides; optionally, by 1 nucleotide).

In the context describing the siRNA antisense strand described herein, the term "a sequence differing from any one of the antisense strands of SEQ ID NO: 31 to SEQ ID NO: 40 by no more than 3 nucleotides and comprises at least 15 contiguous nucleotides" is intended to mean that the siRNA antisense strand described herein comprises at least 15 contiguous nucleotides of any one of the antisense strands of SEQ ID NO: 31 to SEQ ID NO: 40 or a sequence differing from at least 15 contiguous nucleotides of any one of the antisense strands of SEQ ID NO: 31 to SEQ ID NO: 40 by no more than 3 nucleotides (optionally, by no more than 2 nucleotides; optionally, by 1 nucleotide).

In the present disclosure, the "5' region", "5' end", and "5' terminus" of the sense or antisense strand are used interchangeably. For example, the nucleotides at positions 2 to 8 of the 5' region of the antisense strand may also be replaced with the nucleotides at positions 2 to 8 of the 5' end of the antisense strand. Likewise, the "3' region", "3' terminus", and "3' end" of the sense or antisense strand are also used interchangeably.

As used in the present disclosure, the term "2'-fluoro-modified nucleotide/nucleoside" refers to a nucleotide/-nucleoside in which the hydroxy group at the 2' position of the ribosyl group of the nucleotide is substituted with fluorine, and the term "non-2'-fluoro-modified nucleotide/nucleoside" refers to a nucleotide/nucleoside or a nucleotide analog in which the hydroxy group at the 2' position of the ribosyl group of the nucleotide is substituted with a non-fluorine group.

As used in the present disclosure, the term "2'-methoxy-modified nucleotide/nucleoside" refers to a nucleotide/nucleoside in which the 2'-hydroxy group of the ribosyl group is substituted with a methoxy group.

Unless otherwise specified, in the context of the present disclosure, "G", "C", "A", "T", and "U" each represent a nucleotide and contain the bases guanine, cytosine, adenine, thymidine, and uracil, respectively. It is well-known to those

skilled in the art that the mutual replacement between bases T and U does not significantly affect the properties of the siRNA sequence. In the sequences of the present disclosure, U can be arbitrarily replaced with T, and the sequences after the replacement are also within the claimed scope of the present disclosure. In the sequences of the present disclosure, for the same nucleic acid strand, the direction from the 5' end to the 3' end is defined as the direction from the left to the right. The lowercase letter m indicates that the nucleoside adjacent to the left of the letter m is a 2'-methoxy-modified nucleoside; the lowercase letter f indicates that the nucleoside adjacent to the left of the letter f is a 2'-fluoro-modified nucleoside; the lowercase letter s indicates that two nucleosides flanking the letter s are linked by a phosphorothioate diester group. Unless otherwise specified, "RNAi agent", "nucleotide", "compound", "chemical modification", "oligonucleotide", "double-stranded RNAi inhibitor molecule", "siRNA", "siRNA conjugate", "dsRNA", "nucleic acid", and "RNAi" of the present disclosure can each independently be present in the form of a salt, mixed salts, or a non-salt (e.g., a free acid or free base). When being present in the form of a salt or mixed salts, it may be a pharmaceutically acceptable salt. The term "pharmaceutically acceptable salt" includes pharmaceutically acceptable acid addition salts and pharmaceutically acceptable base addition salts. When it is present in the form of a salt, part of the groups may be ionized to form anions/cations; for example, phosphodiester groups and phosphorothioate diester groups may be present in the form of anions, and the structures in the form of a salt corresponding to the following structures are also within the claimed scope of the present disclosure. Unless otherwise specified, the 3' position of the first nucleotide at the 3' end of each strand is hydroxy; the 5' position of the first nucleotide at the 5' end of each strand is hydroxy.

The modifications and linking groups described above separately have the structures shown in the table below, where Base represents a base:

Table 1A

| Structure | Name |
|---|---|
| 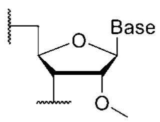 | 2'-Methoxy-modified nucleoside |
| 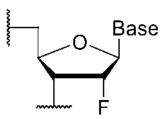 | 2'-Fluoro-modified nucleoside |
| 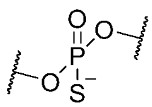 | Anion form of phosphorothioate diester group |
| 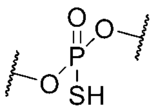 | Phosphorothioate diester group |
| 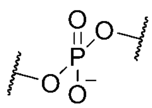 | Anion form of phosphodiester group |
| 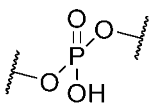 | Phosphodiester group |

As used herein, the terms "complementary" and "reverse complementary" are used interchangeably and have the meaning well-known to those skilled in the art; that is, in a double-stranded nucleic acid molecule, the bases of one strand are paired with the bases of the other strand in a complementary manner. In DNA, the purine base adenine is always paired with the pyrimidine base thymine (or uracil in RNA), and the purine base guanine is always paired with the pyrimidine base cytosine. Each base pair comprises a purine and a pyrimidine. When adenines of one strand are always paired with thymines (or uracils) of another strand and guanines are always paired with cytosines, the two strands are considered complementary to each other, and the sequences of the strands can be deduced from the sequences of their complementary strands. Accordingly, "mismatch" in the art means that in a double-stranded nucleic acid, the bases at the corresponding positions are not paired in a complementary manner.

As used herein, the term "inhibit" is used interchangeably with "decrease", "silence", "down-regulate", "repress", and other similar terms, and includes any level of inhibition. Inhibition can be assessed in terms of a decrease in the absolute or relative level of one or more of these variables relative to a control level. The control level can be any type of control level used in the art, such as a pre-dose baseline level or a level determined from an untreated or control (e.g., buffer-only control or inert agent control) treated subject, cell, or sample. For example, the remaining mRNA expression level can be used to characterize the degree of inhibition of target gene expression by the siRNA; for example, the remaining mRNA expression level is not greater than 99%, not greater than 95%, not greater than 90%, not greater than 85%, not greater than 80%, not greater than 75%, not greater than 70%, not greater than 65%, not greater than 60%, not greater than 55%, not greater than 50%, not greater than 45%, not greater than 40%, not greater than 35%, not greater than 30%, not greater than 25%, not greater than 20%, not greater than 15%, or not greater than 10%. The inhibition rate of target gene expression can be measured using Dual-Glo® Luciferase Assay System: the Firefly chemiluminescence value (Fir) and the Renilla chemiluminescence value (Ren) are each read, and the relative value Ratio = Ren/Fir is calculated. In the present disclosure, the ratio of remaining mRNA expression level (or residual activity%) = Ratio (siRNA-treated group)/Ratio (siRNA-free control group), and inhibition rate (%) = 100% - remaining mRNA expression level (%). Unless otherwise specified, the "compound", "ligand", "nucleic acid-ligand conjugate", "siRNA conjugate", "nucleic acid", "conjugate", "chemical modification", "targeting ligand", "dsRNA", and "RNAi" of the present disclosure can each independently be present in the form of a salt, mixed salts, or a non-salt (e.g., a free acid or free base). When being present in the form of a salt or mixed salts, it may be a pharmaceutically acceptable salt.

The term "pharmaceutically acceptable salt" includes pharmaceutically acceptable acid addition salts and pharmaceutically acceptable base addition salts.

"Pharmaceutically acceptable acid addition salt" refers to salts that are capable of retaining the biological effectiveness of free bases without having any undesirable effects and that are formed with inorganic or organic acids. Inorganic acid salts include, but are not limited to, hydrochlorides, hydrobromides, sulfates, nitrates, phosphates, etc.; organic acid salts include, but are not limited to, formates, acetates, 2,2-dichloroacetates, trifluoroacetates, propionates, caproates, caprylates, caprates, undecenates, glycolates, gluconates, lactates, sebacates, adipates, glutarates, malonates, oxalates, maleates, succinates, fumarates, tartrates, citrates, palmitates, stearates, oleates, cinnamates, laurates, malates, glutamates, pyroglutamates, aspartates, benzoates, mesylates, benzenesulfonates, p-toluenesulfonates, alginates, ascorbates, salicylates, 4-aminosalicylates, napadisylates, etc. These salts can be prepared using methods known in the art.

"Pharmaceutically acceptable base addition salt" refers to salts that are capable of retaining the biological effectiveness of free acids without having any undesirable effects and that are formed with inorganic bases or organic bases. Salts derived from inorganic bases include, but are not limited to, sodium salts, potassium salts, lithium salts, ammonium salts, calcium salts, magnesium salts, iron salts, zinc salts, copper salts, manganese salts, aluminum salts, etc. Preferred inorganic salts are ammonium salts, sodium salts, potassium salts, calcium salts, and magnesium salts; sodium salts are preferred. Salts derived from organic bases include, but are not limited to, salts of the following: primary, secondary, and tertiary amines, substituted amines including naturally occurring substituted amines, cyclic amines, and basic ion exchange resins, such as ammonia, isopropylamine, trimethylamine, diethylamine, triethylamine, tripropylamine, ethanolamine, diethanolamine, triethanolamine, dimethylethanolamine, 2-dimethylaminoethanol, 2-diethylaminoethanol, dicyclohexylamine, lysine, arginine, histidine, caffeine, procaine, choline, betaine, ethylenediamine, glucosamine, methylglucamine, theobromine, purine, piperazine, piperidine, N-ethylpiperidine, polyamine resins, etc. Preferred organic bases include isopropylamine, diethylamine, ethanolamine, trimethylamine, dicyclohexylamine, choline, and caffeine. These salts can be prepared using methods known in the art.

"Effective amount" or "effective dose" refers to the amount of a drug, a compound, or a pharmaceutical composition necessary to obtain any one or more beneficial or desired therapeutic results. For prophylactic use, the beneficial or desired results include elimination or reduction of risk, reduction of severity or delay of the onset of a disorder, including the biochemistry, histology, and/or behavioral symptoms of the disorder, complications thereof, and intermediate pathological phenotypes that appear during the progression of the disorder. For therapeutic applications, the beneficial or desired results include clinical results, such as reducing the incidence of various disorders related to the target gene, target mRNA, or target protein of the present disclosure or ameliorating one or more symptoms of the disorders, reducing the dose of other agents required to treat the disorders, enhancing the therapeutic effect of another agent, and/or delaying the progression of disorders related to the target gene, target mRNA, or target protein of the present disclosure in patients.

As used herein, "patient", "subject", and "individual" are used interchangeably and include human or non-human animals, e.g., mammals, e.g., humans or monkeys.

The siRNA provided by the present disclosure may be obtained using a preparation method conventional in the art (e.g., solid-phase synthesis and liquid-phase synthesis). Solid-phase synthesis has been commercially available as a customization service. A modified nucleotide group can be introduced into the siRNA described in the present disclosure using a nucleoside monomer with a corresponding modification. Methods for preparing the nucleoside monomer with the corresponding modification and introducing the modified nucleotide group into the siRNA are also well-known to those

skilled in the art.

The term "chemical modification" or "modification" includes all changes made to a nucleotide by chemical means, such as the addition or removal of a chemical moiety, or the substitution of one chemical moiety for another.

The term "base" encompasses any known DNA and RNA bases and base analogs such as purines or pyrimidines, and also includes the natural compounds adenine, thymine, guanine, cytosine, uracil, inosine, and natural analogs.

The terms "blunt end" and "blunt ended" are used interchangeably and mean that there are no unpaired nucleotides or nucleotide analogs at a given terminus of an siRNA, i.e., no nucleotide overhang. In most cases, an siRNA, both ends of which are blunt ends, will be double-stranded over its entire length.

The terms "about" and "approximately" mean that a numerical value is within an acceptable margin of error for the specific value determined by those of ordinary skill in the art, and the numerical value depends in part on how the value is measured or determined (i.e., the limitations of the measurement system). For example, "about" may mean a standard deviation within 1 or greater than 1. Alternatively, "about" or "substantially comprise" may mean a range of at most 20%, e.g., a change of between 1% and 15%, between 1% and 10%, between 1% and 5%, between 0.5% and 5%, or between 0.5% and 1%. In the present disclosure, every instance where a number or numerical range is preceded by the term "about" also includes an embodiment of the given number. Unless otherwise specified, when a specific value is provided in the present application and claims, the meaning of "about" or "substantially comprise" should be assumed to be within an acceptable margin of error for that specific value.

Unless otherwise specified, "optionally" or "optional" means that the event or circumstance subsequently described may, but does not necessarily, occur, and this description includes instances where the event or circumstance occurs or does not occur. For example, "optionally, $R_1$ and $R_2$ are directly linked to form a ring" means that $R_1$ and $R_2$ being directly linked to form a ring may occur, but does not necessarily exist, and this description includes an instance where $R_1$ and $R_2$ are directly linked to form a ring and an instance where $R_1$ and $R_2$ do not form a ring.

In the chemical structural formulas of the present disclosure,

“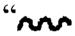” or 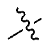

may be linked to any group or groups in accordance with the scope of the invention described herein.

In the context of the present disclosure, the

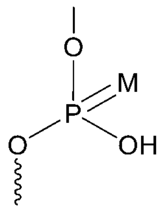

in the group

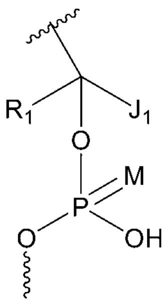

can be replaced with any group capable of linking to an adjacent nucleotide.

The term "link", when referring to a relationship between two molecules, means that the two molecules are linked by a covalent bond or that the two molecules are associated via a non-covalent bond (e.g., a hydrogen bond or an ionic bond), and includes direct linkage and indirect linkage.

The term "directly linked" means that a first compound or group is linked to a second compound or group without any atom or group of atoms interposed between.

The term "indirectly linked" means that a first compound or group is linked to a second compound or group by an

intermediate group, a compound, or a molecule (e.g., a linking group).

The term "substituted" means that any one or more hydrogen atoms on the specified atom (usually a carbon, oxygen, or nitrogen atom) are replaced with any group as defined herein, provided that the normal valency of the specified atom is not exceeded and that the substitution results in a stable compound. Non-limiting examples of substituents include C1-C6 alkyl, C2-C6 alkenyl, C2-C6 alkynyl, cyano, hydroxy, oxo, carboxyl, cycloalkyl, cycloalkenyl, heterocyclyl, heteroaryl, aryl, ketone, alkoxycarbonyl, aryloxycarbonyl, heteroaryloxycarbonyl, or halogen (e.g., F, Cl, Br, or I). When the substituent is a ketone or oxo (i.e., =O), two (2) hydrogens on the atom are replaced.

"Substituted with one or more..." refers to substitution with a single substituent or multiple substituents. In the case of substitution with multiple substituents, there may be a plurality of identical substituents, or one or a group of a plurality of different substituents.

## DETAILED DESCRIPTION

The present disclosure is further described below with reference to examples, but these examples are not intended to limit the scope of the present disclosure. Experimental procedures without specific conditions specified in the examples of the present disclosure were generally conducted under conventional conditions such as those outlined in Antibodies: A Laboratory Manual and Molecular Cloning: A Laboratory Manual by Cold Spring Harbor Laboratory, or under conditions recommended by the manufacturers of the starting materials or commercial products. Reagents without specific origins indicated were commercially available conventional reagents.

### Example 1. Design of Human MMP7 siRNAs

With the human MMP7 gene (NM_002423.5) as a target gene, unmodified or modified 19/21nt siRNAs and siRNA conjugates of the present disclosure were designed in accordance with the general rules for active siRNAs, as shown in Table 1B.

Table 1B. siRNAs and siRNA conjugates targeting human MMP7

| Double strand No. | SEQ ID NO | SS strand (5'-3') | SEQ ID NO | AS strand (5'-3') |
|---|---|---|---|---|
| TJR103296 | 1 | UGGGUAGAUGUCAAUAAAU | 31 | AUUUAUUGACAUCUACCCACU |
| TJR103300 | 2 | UGACUCAGAAACAAAAAAU | 32 | AUUUUUUGUUUCUGAGUCAUA |
| TJR103303 | 3 | AUUCAAGAAAGAAAUAGAA | 33 | UUCUAUUUCUUUCUUGAAUUA |
| TJR103298 | 4 | GACUCAGAAACAAAAAAUA | 34 | UAUUUUUUGUUUCUGAGUCAU |
| TJR103304 | 5 | CAACAGUUUAGAAGCCAAA | 35 | UUUGGCUUCUAAACUGUUGGC |
| TJR103299 | 6 | CAAUUAUGUCACCCUUUUU | 36 | AAAAAGGGUGACAUAAUUGCU |
| TJR103295 | 7 | GUGGGUAGAUGUCAAUAAA | 37 | UUUAUUGACAUCUACCCACUG |
| TJR103301 | 8 | GGGUAGAUGUCAAUAAAUA | 38 | UAUUUAUUGACAUCUACCCAC |
| TJR103302 | 9 | GGUAGAUGUCAAUAAAUGU | 39 | ACAUUUAUUGACAUCUACCCA |
| TJR103297 | 10 | CAAAUAAAUAAAAUGUUUA | 40 | UAAACAUUUUAUUUAUUUGUG |

(continued)

| Double strand No. | SEQ ID NO | SS strand (5'-3') | SEQ ID NO | AS strand (5'-3') |
|---|---|---|---|---|
| TJR101182 | 11 | UmsGmsGmGmUmAmGfAfUfGmUmCmAmAmUmAmAmAmUm | 41 | AmsUfsUmUmAmUfUmGmAmCmAmUfCmUfAmCfCmCmAmsCmsUm |
| TJR101103 | 12 | UmsGmsAmCmUmCmAfGfAfAmAmCmAmAmAmAmAmAmUm | 42 | AmsUfsUmUmUmUfUmGmUmUmUmCfUmGfAmGfUmCmAmsUmsAm |
| TJR101172 | 13 | AmsUmsUmCmAmAmGfAfAfAmGmAmAmAmUmAmGmAmAm | 43 | UmsUfsCmUmAmUfUmUmCmUmUmUfCmUfUmGfAmAmUmsUmsAm |
| TJR101104 | 14 | GmsAmsCmUmCmAmGfAfAfAmCmAmAmAmAmAmAmUmAm | 44 | UmsAfsUmUmUmUfUmUmGmUmUmUfCmUfGmAfGmUmCmsAmsUm |
| TJR103307 | 15 | GmsAmsCmUmCmAmGfAfAfAmCmAmAmAmAmAmAmUmAm | 45 | UmsAfsUmUfUmUfUmUmGmUfUmUfCmUfGmAfGmUfCmsAmsUm |
| TJR101112 | 16 | CmsAmsAmCmAmGmUfUfUfAmGmAmAmGmCmCmAmAmAm | 46 | UmsUfsUmGmGmCfUmUmCmUmAmAfAmCfUmGfUmUmGmsGmsCm |
| TJR103311 | 17 | CmsAmsAmCmAmGmUfUfUfAmGmAmAmGmCmCmAmAmAm | 47 | UmsUfsUmGfGmCfUmUmCmUfAmAfAmCfUmGfUmUfGmsGmsCm |
| TJR101179 | 18 | CmsAmsAmUmUmAmUfGfUfCmAmCmCmCmUmUmUmUmUm | 48 | AmsAfsAmAmAmGfGmGmUmGmAmCfAmUfAmAfUmUmGmsCmsUm |
| TJR103308 | 19 | CmsAmsAmUmUmAmUfGfUfCmAmCmCmCmUmUmUmUmUm | 49 | AmsAfsAmAfAmGfGmGmUmGfAmCfAmUfAmAfUmUfGmsCmsUm |
| TJR101181 | 20 | GmsUmsGmGmGmUmAfGfAfUmGmUmCmAmAmUmAmAmAm | 50 | UmsUfsUmAmUmUfGmAmCmAmUmCfUmAfCmCfCmAmCmsUmsGm |
| TJR103305 | 21 | GmsUmsGmGmGmUmAfGfAfUmGmUmCmAmAmUmAmAmAm | 51 | UmsUfsUmAfUmUfGmAmCmAfUmCfUmAfCmCfCmAfCmsUmsGm |
| TJR101183 | 22 | GmsGmsGmUmAmGmAfUfGfUmCmAmAmUmAmAmAmUmAm | 52 | UmsAfsUmUmUmAfUmUmGmAmCmAfUmCfUmAfCmCmCmsAmsCm |
| TJR103309 | 23 | GmsGmsGmUmAmGmAfUfGfUmCmAmAmUmAmAmAmUmAm | 53 | UmsAfsUmUfUmAfUmUmGmAfCmAfUmCfUmAfCmCfCmsAmsCm |
| TJR101184 | 24 | GmsGmsUmAmGmAmUfGfUfCmAmAmUmAmAmAmUmGmUm | 54 | AmsCfsAmUmUmUfAmUmUmGmAmCfAmUfCmUfAmCmCmsCmsAm |

(continued)

| Double strand No. | SEQ ID NO | SS strand (5'-3') | SEQ ID NO | AS strand (5'-3') |
|---|---|---|---|---|
| TJR103310 | 25 | GmsGmsUmAmGmAmUfGfUfCmAmAmUmAmAmAmUmGmUm | 55 | AmsCfsAmUfUmUfAmUmUmGfAmCfAmUfCmUfAmCfCmsCmsAm |
| TJR101189 | 26 | CmsAmsAmAmUmAmAfAfUfAmAmAmAmUmGmUmUmUmAm | 56 | UmsAfsAmAmCmAfUmUmUmUmAmUfUmUfAmUfUmUmGmsUmsGm |
| TJR103306 | 27 | CmsAmsAmAmUmAmAfAfUfAmAmAmAmUmGmUmUmUmAm | 57 | UmsAfsAmAfCmAfUmUmUmUfAmUfUmUfAmUfUmUfGmsUmsGm |
| TJR103312 | 28 | **I-14**UmsGmsGmGmUmAmGfAfUfGmUmCmAmAmUmAmAmAmUm | 58 | AmsUfsUmUfAmUfUmGmAmCfAmUfCmUfAmCfCmCfAmsCmsUm |
| TJR103313 | 29 | **I-14**UmsGmsAmCmUmCmAfGfAfAmAmCmAmAmAmAmAmAmUm | 59 | AmsUfsUmUfUmUfUmGmUmUfUmCfUmGfAmGfUmCfAmsUmsAm |
| TJR103314 | 30 | **I-14**AmsUmsUmCmAmAmGfAfAfAmGmAmAmAmUmAmGmAmAm | 60 | UmsUfsCmUfAmUfUmUmCmUfUmUfCmUfUmGfAmAfUmsUmsAm |
| | 61 | CAAAUCAACCAUAGGUCCA | 152 | UGGACCUAUGGUUGAUUUGGU |
| | 62 | AACCAUAGGUCCAAGAACA | 153 | UGUUCUUGGACCUAUGGUUGA |
| | 63 | ACCAUAGGUCCAAGAACAA | 154 | UUGUUCUUGGACCUAUGGUUG |
| | 64 | CCAUAGGUCCAAGAACAAU | 155 | AUUGUUCUUGGACCUAUGGUU |
| | 65 | CAUAGGUCCAAGAACAAUU | 156 | AAUUGUUCUUGGACCUAUGGU |
| | 66 | GGUCCAAGAACAAUUGUCU | 157 | AGACAAUUGUUCUUGGACCUA |
| | 67 | GGGAGGCAUGAGUGAGCUA | 158 | UAGCUCACUCAUGCCUCCCGC |
| | 68 | AGGACUAUCUCAAGAGAUU | 159 | AAUCUCUUGAGAUAGUCCUGA |
| | 69 | GGACUAUCUCAAGAGAUUU | 160 | AAAUCUCUUGAGAUAGUCCUG |
| | 70 | GACUAUCUCAAGAGAUUUU | 161 | AAAAUCUCUUGAGAUAGUCCU |

(continued)

| Double strand No. | SEQ ID NO | SS strand (5'-3') | SEQ ID NO | AS strand (5'-3') |
|---|---|---|---|---|
| | 71 | CUCUAUGACUCAGAAACAA | 162 | UUGUUUCUGAGUCAUAGAGAU |
| | 72 | CUAUGACUCAGAAACAAAA | 163 | UUUUGUUUCUGAGUCAUAGAG |
| | 73 | CAGAAACAAAAAAUGCCAA | 164 | UUGGCAUUUUUUGUUUCUGAG |
| | 74 | GAAACAAAAAAUGCCAACA | 165 | UGUUGGCAUUUUUUGUUUCUG |
| | 75 | CAAAAAAUGCCAACAGUUU | 166 | AAACUGUUGGCAUUUUUUGUU |
| | 76 | AAAAAAUGCCAACAGUUUA | 167 | UAAACUGUUGGCAUUUUUUGU |
| | 77 | AAAUGCCAACAGUUUAGAA | 168 | UUCUAAACUGUUGGCAUUUUU |
| | 78 | GCCAACAGUUUAGAAGCCA | 169 | UGGCUUCUAAACUGUUGGCAU |
| | 79 | CCAACAGUUUAGAAGCCAA | 170 | UUGGCUUCUAAACUGUUGGCA |
| | 80 | AGUUUAGAAGCCAAACUCA | 171 | UGAGUUUGGCUUCUAAACUGU |
| | 81 | GUUUAGAAGCCAAACUCAA | 172 | UUGAGUUUGGCUUCUAAACUG |
| | 82 | GAAGCCAAACUCAAGGAGA | 173 | UCUCCUUGAGUUUGGCUUCUA |
| | 83 | GAAAUAAUGCAGAAGCCCA | 174 | UGGGCUUCUGCAUUAUUUCUA |
| | 84 | GCAGAAGCCCAGAUGUGGA | 175 | UCCACAUCUGGGCUUCUGCAU |
| | 85 | GCCCAGAUGUGGAGUGCCA | 176 | UGGCACUCCACAUCUGGGCUU |
| | 86 | AAUACUCACUAUUUCCAAA | 177 | UUUGGAAAUAGUGAGUAUUCU |
| | 87 | UACUCACUAUUUCCAAAUA | 178 | UAUUUGGAAAUAGUGAGUAUU |
| | 88 | CUAUUUCCAAAUAGCCCAA | 179 | UUGGGCUAUUUGGAAAUAGUG |
| | 89 | CCAAAUAGCCCAAAAUGGA | 180 | UCCAUUUUGGGCUAUUUGGAA |

(continued)

| Double strand No. | SEQ ID NO | SS strand (5'-3') | SEQ ID NO | AS strand (5'-3') |
|---|---|---|---|---|
| | 90 | AGCCCAAAAUGGACUUCCA | 181 | UGGAAGUCCAUUUUGGGCUAU |
| | 91 | GCCCAAAAUGGACUUCCAA | 182 | UUGGAAGUCCAUUUUGGGCUA |
| | 92 | CCCAAAAUGGACUUCCAAA | 183 | UUUGGAAGUCCAUUUUGGGCU |
| | 93 | AUCAUAUACUCGAGACUUA | 184 | UAAGUCUCGAGUAUAUGAUAC |
| | 94 | ACUCGAGACUUACCGCAUA | 185 | UAUGCGGUAAGUCUCGAGUAU |
| | 95 | CGAUUAGUGUCAAAGGCUU | 186 | AAGCCUUUGACACUAAUCGAU |
| | 96 | GAUUAGUGUCAAAGGCUUU | 187 | AAAGCCUUUGACACUAAUCGA |
| | 97 | GUGUCAAAGGCUUUAAACA | 188 | UGUUUAAAGCCUUUGACACUA |
| | 98 | UGUCAAAGGCUUUAAACAU | 189 | AUGUUUAAAGCCUUUGACACU |
| | 99 | GCAAAGAGAUCCCCCUGCA | 190 | UGCAGGGGGAUCUCUUUGCCC |
| | 100 | GAGAUCCCCCUGCAUUUCA | 191 | UGAAAUGCAGGGGGAUCUCUU |
| | 101 | CCCCCUGCAUUUCAGGAAA | 192 | UUUCCUGAAAUGCAGGGGGAU |
| | 102 | CCUGCAUUUCAGGAAAGUU | 193 | AACUUUCCUGAAAUGCAGGGG |
| | 103 | GCAUUUCAGGAAAGUUGUA | 194 | UACAACUUUCCUGAAAUGCAG |
| | 104 | CAGGAAAGUUGUAUGGGGA | 195 | UCCCCAUACAACUUUCCUGAA |
| | 105 | AGGAAAGUUGUAUGGGGAA | 196 | UUCCCCAUACAACUUUCCUGA |
| | 106 | GGGGAACUGCUGACAUCAU | 197 | AUGAUGUCAGCAGUUCCCCAU |
| | 107 | GGAACUGCUGACAUCAUGA | 198 | UCAUGAUGUCAGCAGUUCCCC |
| | 108 | GAACUGCUGACAUCAUGAU | 199 | AUCAUGAUGUCAGCAGUUCCC |

(continued)

| Double strand No. | SEQ ID NO | SS strand (5'-3') | SEQ ID NO | AS strand (5'-3') |
|---|---|---|---|---|
| | 109 | CUGACAUCAUGAUUGGCUU | 200 | AAGCCAAUCAUGAUGUCAGCA |
| | 110 | CAGGAAACACGCUGGCUCA | 201 | UGAGCCAGCGUGUUUCCUGGC |
| | 111 | CACGCUGGCUCAUGCCUUU | 202 | AAAGGCAUGAGCCAGCGUGUU |
| | 112 | UCACUUCGAUGAGGAUGAA | 203 | UUCAUCCUCAUCGAAGUGAGC |
| | 113 | GGAUGAACGCUGGACGGAU | 204 | AUCCGUCCAGCGUUCAUCCUC |
| | 114 | GACGGAUGGUAGCAGUCUA | 205 | UAGACUGCUACCAUCCGUCCA |
| | 115 | AGAUCCCCAAAAUUUUAAA | 206 | UUUAAAAUUUUGGGGAUCUCC |
| | 116 | CCCCAAAAUUUUAAACUUU | 207 | AAAGUUUAAAAUUUUGGGGAU |
| | 117 | CCCAAAAUUUUAAACUUUA | 208 | UAAAGUUUAAAAUUUUGGGGA |
| | 118 | AAACUUUCCCAGGAUGAUA | 209 | UAUCAUCCUGGGAAAGUUUAA |
| | 119 | GAUAUUAAAGGCAUUCAGA | 210 | UCUGAAUGCCUUUAAUAUCAU |
| | 120 | AUAUUAAAGGCAUUCAGAA | 211 | UUCUGAAUGCCUUUAAUAUCA |
| | 121 | UAUUAAAGGCAUUCAGAAA | 212 | UUUCUGAAUGCCUUUAAUAUC |
| | 122 | UUCAGAAACUAUAUGGAAA | 213 | UUUCCAUAUAGUUUCUGAAUG |
| | 123 | CAGAAACUAUAUGGAAAGA | 214 | UCUUUCCAUAUAGUUUCUGAA |
| | 124 | GAAACUAUAUGGAAAGAGA | 215 | UCUCUUUCCAUAUAGUUUCUG |
| | 125 | AAACUAUAUGGAAAGAGAA | 216 | UUCUCUUUCCAUAUAGUUUCU |
| | 126 | CUAUAUGGAAAGAGAAGUA | 217 | UACUUCUCUUUCCAUAUAGUU |
| | 127 | AUAUGGAAAGAGAAGUAAU | 218 | AUUACUUCUCUUUCCAUAUAG |

(continued)

| Double strand No. | SEQ ID NO | SS strand (5'-3') | SEQ ID NO | AS strand (5'-3') |
|---|---|---|---|---|
| | 128 | UGGAAAGAGAAGUAAUUCA | 219 | UGAAUUACUUCUCUUUCCAUA |
| | 129 | GGAAAGAGAAGUAAUUCAA | 220 | UUGAAUUACUUCUCUUUCCAU |
| | 130 | AAAGAGAAGUAAUUCAAGA | 221 | UCUUGAAUUACUUCUCUUUCC |
| | 131 | AAGAGAAGUAAUUCAAGAA | 222 | UUCUUGAAUUACUUCUCUUUC |
| | 132 | AGAGAAGUAAUUCAAGAAA | 223 | UUUCUUGAAUUACUUCUCUUU |
| | 133 | GAGAAGUAAUUCAAGAAAA | 224 | UUUUCUUGAAUUACUUCUCUU |
| | 134 | AGAAGUAAUUCAAGAAAGA | 225 | UCUUUCUUGAAUUACUUCUCU |
| | 135 | GAAGUAAUUCAAGAAAGAA | 226 | UUCUUUCUUGAAUUACUUCUC |
| | 136 | AAGUAAUUCAAGAAAGAAA | 227 | UUUCUUUCUUGAAUUACUUCU |
| | 137 | GUAAUUCAAGAAAGAAAUA | 228 | UAUUUCUUUCUUGAAUUACUU |
| | 138 | AAUUCAAGAAAGAAAUAGA | 229 | UCUAUUUCUUUCUUGAAUUAC |
| | 139 | CAAGAAAGAAAUAGAAACU | 230 | AGUUUCUAUUUCUUUCUUGAA |
| | 140 | AGAAAGAAAUAGAAACUUA | 231 | UAAGUUUCUAUUUCUUUCUUG |
| | 141 | GAAAGAAAUAGAAACUUCA | 232 | UGAAGUUUCUAUUUCUUUCUU |
| | 142 | UAUCAUUGUUGCACAAUCA | 233 | UGAUUGUGCAACAAUGAUAUA |
| | 143 | UCAUUGUUGCACAAUCAGA | 234 | UCUGAUUGUGCAACAAUGAUA |
| | 144 | GCAAUUAUGUCACCCUUUU | 235 | AAAAGGGUGACAUAAUUGCUA |
| | 145 | GAAUGUCUUUCACUCCUUU | 236 | AAAGGAGUGAAAGACAUUCAA |
| | 146 | GUAGAUGUCAAUAAAUGUU | 237 | AACAUUUAUUGACAUCUACCC |

(continued)

| Double strand No. | SEQ ID NO | SS strand (5'-3') | SEQ ID NO | AS strand (5'-3') |
|---|---|---|---|---|
| | 147 | AGAUGUCAAUAAAUGUUAA | 238 | UUAACAUUUAUUGACAUCUAC |
| | 148 | CACAAAUAAAUAAAAUGUU | 239 | AACAUUUUAUUUAUUUGUGUA |
| | 149 | ACAAAUAAAUAAAAUGUUU | 240 | AAACAUUUUAUUUAUUUGUGU |
| | 150 | AAAUAAAUAAAAUGUUUAU | 241 | AUAAACAUUUUAUUUAUUUGU |
| | 151 | AAAUAAAAUGUUUAUUCCA | 242 | UGGAAUAAACAUUUUAUUUAU |
| TJR101091 | 243 | CmsAmsAmAmUmCmAfAfCfCmAmUmAmGmGmUmCmCmAm | 334 | UmsGfsGmAmCmCfUmAmUmGmGmUfUmGfAmUfUmUmGmsGmsUm |
| TJR101092 | 244 | AmsAmsCmCmAmUmAfGfGfUmCmCmAmAmGmAmAmCmAm | 335 | UmsGfsUmUmCmUfUmGmGmAmCmCfUmAfUmGfGmUmUmsGmsAm |
| TJR101093 | 245 | AmsCmsCmAmUmAmGfGfUfCmCmAmAmGmAmAmCmAmAm | 336 | UmsUfsGmUmUmCfUmUmGmGmAmCfCmUfAmUfGmGmUmsUmsGm |
| TJR101094 | 246 | CmsCmsAmUmAmGmGfUfCfCmAmAmGmAmAmCmAmAmUm | 337 | AmsUfsUmGmUmUfCmUmUmGmGmAfCmCfUmAfUmGmGmsUmsUm |
| TJR101095 | 247 | CmsAmsUmAmGmGmUfCfCfAmAmGmAmAmCmAmAmUmUm | 338 | AmsAfsUmUmGmUfUmCmUmUmGmGfAmCfCmUfAmUmGmsGmsUm |
| TJR101096 | 248 | GmsGmsUmCmCmAmAfGfAfAmCmAmAmUmUmGmUmCmUm | 339 | AmsGfsAmCmAmAfUmUmGmUmUmCfUmUfGmGfAmCmCmsUmsAm |
| TJR101097 | 249 | GmsGmsGmAmGmGmCfAfUfGmAmGmUmGmAmGmCmUmAm | 340 | UmsAfsGmCmUmCfAmCmUmCmAmUfGmCfCmUfCmCmCmsGmsCm |
| TJR101098 | 250 | AmsGmsGmAmCmUmAfUfCfUmCmAmAmGmAmGmAmUmUm | 341 | AmsAfsUmCmUmCfUmUmGmAmGmAfUmAfGmUfCmCmUmsGmsAm |
| TJR101099 | 251 | GmsGmsAmCmUmAmUfCfUfCmAmAmGmAmGmAmUmUmUm | 342 | AmsAfsAmUmCmUfCmUmUmGmAmGfAmUfAmGfUmCmCmsUmsGm |
| TJR101100 | 252 | GmsAmsCmUmAmUmCfUfCfAmAmGmAmGmAmUmUmUmUm | 343 | AmsAfsAmAmUmCfUmCmUmUmGmAfGmAfUmAfGmUmCmsCmsUm |
| TJR101101 | 253 | CmsUmsCmUmAmUmGfAfCfUmCmAmGmAmAmAmCmAmAm | 344 | UmsUfsGmUmUmUfCmUmGmAmGmUfCmAfUmAfGmAmGmsAmsUm |

(continued)

| Double strand No. | SEQ ID NO | SS strand (5'-3') | SEQ ID NO | AS strand (5'-3') |
|---|---|---|---|---|
| TJR101102 | 254 | CmsUmsAmUmGmAmCfUfCfAmGmAmAmAmCmAmAmAmAm | 345 | UmsUfsUmUmGmUfUmUmCmUmGmAfGmUfCmAfUmAmGmsAmsGm |
| TJR101105 | 255 | CmsAmsGmAmAmAmCfAfAfAmAmAmAmUmGmCmCmAmAm | 346 | UmsUfsGmGmCmAfUmUmUmUmUmUfGmUfUmUfCmUmGmsAmsGm |
| TJR101106 | 256 | GmsAmsAmAmCmAmAfAfAfAmAmUmGmCmCmAmAmCmAm | 347 | UmsGfsUmUmGmGfCmAmUmUmUmUfUmUfGmUfUmUmCmsUmsGm |
| TJR101107 | 257 | CmsAmsAmAmAmAmAfUfGfCmCmAmAmCmAmGmUmUmUm | 348 | AmsAfsAmCmUmGfUmUmGmGmCmAfUmUfUmUfUmUmGmsUmsUm |
| TJR101108 | 258 | AmsAmsAmAmAmAmUfGfCfCmAmAmCmAmGmUmUmUmAm | 349 | UmsAfsAmAmCmUfGmUmUmGmGmCfAmUfUmUfUmUmUmsGmsUm |
| TJR101109 | 259 | AmsAmsAmUmGmCmCfAfAfCmAmGmUmUmUmAmGmAmAm | 350 | UmsUfsCmUmAmAfAmCmUmGmUmUfGmGfCmAfUmUmUmsUmsUm |
| TJR101110 | 260 | GmsCmsCmAmAmCmAfGfUfUmUmAmGmAmAmGmCmCmAm | 351 | UmsGfsGmCmUmUfCmUmAmAmAmCfUmGfUmUfGmGmCmsAmsUm |
| TJR101111 | 261 | CmsCmsAmAmCmAmGfUfUfUmAmGmAmAmGmCmCmAmAm | 352 | UmsUfsGmGmCmUfUmCmUmAmAmAfCmUfGmUfUmGmGmsCmsAm |
| TJR101113 | 262 | AmsGmsUmUmUmAmGfAfAfGmCmCmAmAmAmCmUmCmAm | 353 | UmsGfsAmGmUmUfUmGmGmCmUmUfCmUfAmAfAmCmUmsGmsUm |
| TJR101114 | 263 | GmsUmsUmUmAmGmAfAfGfCmCmAmAmAmCmUmCmAmAm | 354 | UmsUfsGmAmGmUfUmUmGmGmCmUfUmCfUmAfAmAmCmsUmsGm |
| TJR101115 | 264 | GmsAmsAmGmCmCmAfAfAfCmUmCmAmAmGmGmAmGmAm | 355 | UmsCfsUmCmCmUfUmGmAmGmUmUfUmGfGmCfUmUmCmsUmsAm |
| TJR101116 | 265 | GmsAmsAmAmUmAmAfUfGfCmAmGmAmAmGmCmCmCmAm | 356 | UmsGfsGmGmCmUfUmCmUmGmCmAfUmUfAmUfUmUmCmsUmsAm |
| TJR101117 | 266 | GmsCmsAmGmAmAmGfCfCfCmAmGmAmUmGmUmGmGmAm | 357 | UmsCfsCmAmCmAfUmCmUmGmGmGfCmUfUmCfUmGmCmsAmsUm |
| TJR101118 | 267 | GmsCmsCmCmAmGmAfUfGfUmGmGmAmGmUmGmCmCmAm | 358 | UmsGfsGmCmAmCfUmCmCmAmCmAfUmCfUmGfGmGmCmsUmsUm |

(continued)

| Double strand No. | SEQ ID NO | SS strand (5'-3') | SEQ ID NO | AS strand (5'-3') |
|---|---|---|---|---|
| TJR101119 | 268 | AmsAmsUmAmCmUmCfAfCfUmAmUmUmUmCmCmAmAmAm | 359 | UmsUfsUmGmGmAfAmAmUmAmGmUfGmAfGmUfAmUmUmsCmsUm |
| TJR101120 | 269 | UmsAmsCmUmCmAmCfUfAfUmUmUmCmCmAmAmAmUmAm | 360 | UmsAfsUmUmUmGfGmAmAmAmUmAfGmUfGmAfGmUmAmsUmsUm |
| TJR101121 | 270 | CmsUmsAmUmUmUmCfCfAfAmAmUmAmGmCmCmCmAmAm | 361 | UmsUfsGmGmGmCfUmAmUmUmUmGfGmAfAmAfUmAmGmsUmsGm |
| TJR101122 | 271 | CmsCmsAmAmAmUmAfGfCfCmCmAmAmAmAmUmGmGmAm | 362 | UmsCfsCmAmUmUfUmUmGmGmGmCfUmAfUmUfUmGmGmsAmsAm |
| TJR101123 | 272 | AmsGmsCmCmCmAmAfAfAfUmGmGmAmCmUmUmCmCmAm | 363 | UmsGfsGmAmAmGfUmCmCmAmUmUfUmUfGmGfGmCmUmsAmsUm |
| TJR101124 | 273 | GmsCmsCmCmAmAmAfAfUfGmGmAmCmUmUmCmCmAmAm | 364 | UmsUfsGmGmAmAfGmUmCmCmAmUfUmUfUmGfGmGmCmsUmsAm |
| TJR101125 | 274 | CmsCmsCmAmAmAmAfUfGfGmAmCmUmUmCmCmAmAmAm | 365 | UmsUfsUmGmGmAfAmGmUmCmCmAfUmUfUmUfGmGmGmsCmsUm |
| TJR101126 | 275 | AmsUmsCmAmUmAmUfAfCfUmCmGmAmGmAmCmUmUmAm | 366 | UmsAfsAmGmUmCfUmCmGmAmGmUfAmUfAmUfGmAmUmsAmsCm |
| TJR101127 | 276 | AmsCmsUmCmGmAmGfAfCfUmUmAmCmCmGmCmAmUmAm | 367 | UmsAfsUmGmCmGfGmUmAmAmGmUfCmUfCmGfAmGmUmsAmsUm |
| TJR101128 | 277 | CmsGmsAmUmUmAmGfUfGfUmCmAmAmAmGmGmCmUmUm | 368 | AmsAfsGmCmCmUfUmUmGmAmCmAfCmUfAmAfUmCmGmsAmsUm |
| TJR101129 | 278 | GmsAmsUmUmAmGmUfGfUfCmAmAmAmGmGmCmUmUmUm | 369 | AmsAfsAmGmCmCfUmUmUmGmAmCfAmCfUmAfAmUmCmsGmsAm |
| TJR101130 | 279 | GmsUmsGmUmCmAmAfAfGfGmCmUmUmUmAmAmAmCmAm | 370 | UmsGfsUmUmUmAfAmAmGmCmCmUfUmUfGmAfCmAmCmsUmsAm |
| TJR101131 | 280 | UmsGmsUmCmAmAmAfGfGfCmUmUmUmAmAmAmCmAmUm | 371 | AmsUfsGmUmUmUfAmAmAmGmCmCfUmUfUmGfAmCmAmsCmsUm |
| TJR101132 | 281 | GmsCmsAmAmAmGmAfGfAfUmCmCmCmCmCmUmGmCmAm | 372 | UmsGfsCmAmGmGfGmGmGmAmUmCfUmCfUmUfUmGmCmsCmsCm |

(continued)

| Double strand No. | SEQ ID NO | SS strand (5'-3') | SEQ ID NO | AS strand (5'-3') |
|---|---|---|---|---|
| TJR101133 | 282 | GmsAmsGmAmUmCmCfCfCfCmUmGmCmAmUmUmUmCmAm | 373 | UmsGfsAmAmAmUfGmCmAmGmGmGfGmGfAmUfCmUmCmsUmsUm |
| TJR101134 | 283 | CmsCmsCmCmCmUmGfCfAfUmUmUmCmAmGmGmAmAmAm | 374 | UmsUfsUmCmCmUfGmAmAmAmUmGfCmAfGmGfGmGmGmsAmsUm |
| TJR101135 | 284 | CmsCmsUmGmCmAmUfUfUfCmAmGmGmAmAmAmGmUmUm | 375 | AmsAfsCmUmUmUfCmCmUmGmAmAfAmUfGmCfAmGmGmsGmsGm |
| TJR101136 | 285 | GmsCmsAmUmUmUmCfAfGfGmAmAmAmGmUmUmGmUmAm | 376 | UmsAfsCmAmAmCfUmUmUmCmCmUfGmAfAmAfUmGmCmsAmsGm |
| TJR101137 | 286 | CmsAmsGmGmAmAmAfGfUfUmGmUmAmUmGmGmGmGmAm | 377 | UmsCfsCmCmCmAfUmAmCmAmAmCfUmUfUmCfCmUmGmsAmsAm |
| TJR101138 | 287 | AmsGmsGmAmAmAmGfUfUfGmUmAmUmGmGmGmGmAmAm | 378 | UmsUfsCmCmCmCfAmUmAmCmAmAfCmUfUmUfCmCmUmsGmsAm |
| TJR101139 | 288 | GmsGmsGmGmAmAmCfUfGfCmUmGmAmCmAmUmCmAmUm | 379 | AmsUfsGmAmUmGfUmCmAmGmCmAfGmUfUmCfCmCmCmsAmsUm |
| TJR101140 | 289 | GmsGmsAmAmCmUmGfCfUfGmAmCmAmUmCmAmUmGmAm | 380 | UmsCfsAmUmGmAfUmGmUmCmAmGfCmAfGmUfUmCmCmsCmsCm |
| TJR101141 | 290 | GmsAmsAmCmUmGmCfUfGfAmCmAmUmCmAmUmGmAmUm | 381 | AmsUfsCmAmUmGfAmUmGmUmCmAfGmCfAmGfUmUmCmsCmsCm |
| TJR101142 | 291 | CmsUmsGmAmCmAmUfCfAfUmGmAmUmUmGmGmCmUmUm | 382 | AmsAfsGmCmCmAfAmUmCmAmUmGfAmUfGmUfCmAmGmsCmsAm |
| TJR101143 | 292 | CmsAmsGmGmAmAmAfCfAfCmGmCmUmGmGmCmUmCmAm | 383 | UmsGfsAmGmCmCfAmGmCmGmUmGfUmUfUmCfCmUmGmsGmsCm |
| TJR101144 | 293 | CmsAmsCmGmCmUmGfGfCfUmCmAmUmGmCmCmUmUmUm | 384 | AmsAfsAmGmGmCfAmUmGmAmGmCfCmAfGmCfGmUmGmsUmsUm |
| TJR101145 | 294 | UmsCmsAmCmUmUmCfGfAfUmGmAmGmGmAmUmGmAmAm | 385 | UmsUfsCmAmUmCfCmUmCmAmUmCfGmAfAmGfUmGmAmsGmsCm |
| TJR101146 | 295 | GmsGmsAmUmGmAmAfCfGfCmUmGmGmAmCmGmGmAmUm | 386 | AmsUfsCmCmGmUfCmCmAmGmCmGfUmUfCmAfUmCmCmsUmsCm |

(continued)

| Double strand No. | SEQ ID NO | SS strand (5'-3') | SEQ ID NO | AS strand (5'-3') |
|---|---|---|---|---|
| TJR101147 | 296 | GmsAmsCmGmGmAmUfGfGfUmAmGmCmAmGmUmCmUmAm | 387 | UmsAfsGmAmCmUfGmCmUmAmCmCfAmUfCmCfGmUmCmsCmsAm |
| TJR101148 | 297 | AmsGmsAmUmCmCmCfCfAfAmAmAmUmUmUmUmAmAmAm | 388 | UmsUfsUmAmAmAfAmUmUmUmUmGfGmGfGmAfUmCmUmsCmsCm |
| TJR101149 | 298 | CmsCmsCmCmAmAmAfAfUfUmUmUmAmAmAmCmUmUmUm | 389 | AmsAfsAmGmUmUfUmAmAmAmAmUfUmUfUmGfGmGmGmsAmsUm |
| TJR101150 | 299 | CmsCmsCmAmAmAmAfUfUfUmUmAmAmAmCmUmUmUmAm | 390 | UmsAfsAmAmGmUfUmUmAmAmAmAfUmUfUmUfGmGmGmsGmsAm |
| TJR101151 | 300 | AmsAmsAmCmUmUmUfCfCfCmAmGmGmAmUmGmAmUmAm | 391 | UmsAfsUmCmAmUfCmCmUmGmGmGfAmAfAmGfUmUmUmsAmsAm |
| TJR101152 | 301 | GmsAmsUmAmUmUmAfAfAfGmGmCmAmUmUmCmAmGmAm | 392 | UmsCfsUmGmAmAfUmGmCmCmUmUfUmAfAmUfAmUmCmsAmsUm |
| TJR101153 | 302 | AmsUmsAmUmUmAmAfAfGfGmCmAmUmUmCmAmGmAmAm | 393 | UmsUfsCmUmGmAfAmUmGmCmCmUfUmUfAmAfUmAmUmsCmsAm |
| TJR101154 | 303 | UmsAmsUmUmAmAmAfGfGfCmAmUmUmCmAmGmAmAmAm | 394 | UmsUfsUmCmUmGfAmAmUmGmCmCfUmUfUmAfAmUmAmsUmsCm |
| TJR101155 | 304 | UmsUmsCmAmGmAmAfAfCfUmAmUmAmUmGmGmAmAmAm | 395 | UmsUfsUmCmCmAfUmAmUmAmGmUfUmUfCmUfGmAmAmsUmsGm |
| TJR101156 | 305 | CmsAmsGmAmAmAmCfUfAfUmAmUmGmGmAmAmAmGmAm | 396 | UmsCfsUmUmUmCfCmAmUmAmUmAfGmUfUmUfCmUmGmsAmsAm |
| TJR101157 | 306 | GmsAmsAmAmCmUmAfUfAfUmGmGmAmAmAmGmAmGmAm | 397 | UmsCfsUmCmUmUfUmCmCmAmUmAfUmAfGmUfUmUmCmsUmsGm |
| TJR101158 | 307 | AmsAmsAmCmUmAmUfAfUfGmGmAmAmAmGmAmGmAmAm | 398 | UmsUfsCmUmCmUfUmUmCmCmAmUfAmUfAmGfUmUmUmsCmsUm |
| TJR101159 | 308 | CmsUmsAmUmAmUmGfGfAfAmAmGmAmGmAmAmGmUmAm | 399 | UmsAfsCmUmUmCfUmCmUmUmUmCfCmAfUmAfUmAmGmsUmsUm |
| TJR101160 | 309 | AmsUmsAmUmGmGmAfAfAfGmAmGmAmAmGmUmAmAmUm | 400 | AmsUfsUmAmCmUfUmCmUmCmUmUfUmCfCmAfUmAmUmsAmsGm |

(continued)

| Double strand No. | SEQ ID NO | SS strand (5'-3') | SEQ ID NO | AS strand (5'-3') |
|---|---|---|---|---|
| TJR101161 | 310 | UmsGmsGmAmAmAmGfAfGfAmAmGmUmAmAmUmUmCmAm | 401 | UmsGfsAmAmUmUfAmCmUmUmCmUfCmUfUmUfCmCmAmsUmsAm |
| TJR101162 | 311 | GmsGmsAmAmAmGmAfGfAfAmGmUmAmAmUmUmCmAmAm | 402 | UmsUfsGmAmAmUfUmAmCmUmUmCfUmCfUmUfUmCmCmsAmsUm |
| TJR101163 | 312 | AmsAmsAmGmAmGmAfAfGfUmAmAmUmUmCmAmAmGmAm | 403 | UmsCfsUmUmGmAfAmUmUmAmCmUfUmCfUmCfUmUmUmsCmsCm |
| TJR101164 | 313 | AmsAmsGmAmGmAmAfGfUfAmAmUmUmCmAmAmGmAmAm | 404 | UmsUfsCmUmUmGfAmAmUmUmAmCfUmUfCmUfCmUmUmsUmsCm |
| TJR101165 | 314 | AmsGmsAmGmAmAmGfUfAfAmUmUmCmAmAmGmAmAmAm | 405 | UmsUfsUmCmUmUfGmAmAmUmUmAfCmUfUmCfUmCmUmsUmsUm |
| TJR101166 | 315 | GmsAmsGmAmAmGmUfAfAfUmUmCmAmAmGmAmAmAmAm | 406 | UmsUfsUmUmCmUfUmGmAmAmUmUfAmCfUmUfCmUmCmsUmsUm |
| TJR101167 | 316 | AmsGmsAmAmGmUmAfAfUfUmCmAmAmGmAmAmAmGmAm | 407 | UmsCfsUmUmUmCfUmUmGmAmAmUfUmAfCmUfUmCmUmsCmsUm |
| TJR101168 | 317 | GmsAmsAmGmUmAmAfUfUfCmAmAmGmAmAmAmGmAmAm | 408 | UmsUfsCmUmUmUfCmUmUmGmAmAfUmUfAmCfUmUmCmsUmsCm |
| TJR101169 | 318 | AmsAmsGmUmAmAmUfUfCfAmAmGmAmAmAmGmAmAmAm | 409 | UmsUfsUmCmUmUfUmCmUmUmGmAfAmUfUmAfCmUmUmsCmsUm |
| TJR101170 | 319 | GmsUmsAmAmUmUmCfAfAfGmAmAmAmGmAmAmAmUmAm | 410 | UmsAfsUmUmUmCfUmUmUmCmUmUfGmAfAmUfUmAmCmsUmsUm |
| TJR101171 | 320 | AmsAmsUmUmCmAmAfGfAfAmAmGmAmAmAmUmAmGmAm | 411 | UmsCfsUmAmUmUfUmCmUmUmUmCfUmUfGmAfAmUmUmsAmsCm |
| TJR101173 | 321 | CmsAmsAmGmAmAmAfGfAfAmAmUmAmGmAmAmAmCmUm | 412 | AmsGfsUmUmUmCfUmAmUmUmUmCfUmUfUmCfUmUmGmsAmsAm |
| TJR101174 | 322 | AmsGmsAmAmAmGmAfAfAfUmAmGmAmAmAmCmUmUmAm | 413 | UmsAfsAmGmUmUfUmCmUmAmUmUfUmCfUmUfUmCmUmsUmsGm |
| TJR101175 | 323 | GmsAmsAmAmGmAmAfAfUfAmGmAmAmAmCmUmUmCmAm | 414 | UmsGfsAmAmGmUfUmUmCmUmAmUfUmUfCmUfUmUmCmsUmsUm |

(continued)

| Double strand No. | SEQ ID NO | SS strand (5'-3') | SEQ ID NO | AS strand (5'-3') |
|---|---|---|---|---|
| TJR101176 | 324 | UmsAmsUmCmAmUmUfGfUfUmGmCmAmCmAmAmUmCmAm | 415 | UmsGfsAmUmUmGfUmGmCmAmAmCfAmAfUmGfAmUmAmsUmsAm |
| TJR101177 | 325 | UmsCmsAmUmUmGmUfUfGfCmAmCmAmAmUmCmAmGmAm | 416 | UmsCfsUmGmAmUfUmGmUmGmCmAfAmCfAmAfUmGmAmsUmsAm |
| TJR101178 | 326 | GmsCmsAmAmUmUmAfUfGfUmCmAmCmCmCmUmUmUmUm | 417 | AmsAfsAmAmGmGfGmUmGmAmCmAfUmAfAmUfUmGmCmsUmsAm |
| TJR101180 | 327 | GmsAmsAmUmGmUmCfUfUfUmCmAmCmUmCmCmUmUmUm | 418 | AmsAfsAmGmGmAfGmUmGmAmAmAfGmAfCmAfUmUmCmsAmsAm |
| TJR101185 | 328 | GmsUmsAmGmAmUmGfUfCfAmAmUmAmAmAmUmGmUmUm | 419 | AmsAfsCmAmUmUfUmAmUmUmGmAfCmAfUmCfUmAmCmsCmsCm |
| TJR101186 | 329 | AmsGmsAmUmGmUmCfAfAfUmAmAmAmUmGmUmUmAmAm | 420 | UmsUfsAmAmCmAfUmUmUmAmUmUfGmAfCmAfUmCmUmsAmsCm |
| TJR101187 | 330 | CmsAmsCmAmAmAmUfAfAfAmUmAmAmAmAmUmGmUmUm | 421 | AmsAfsCmAmUmUfUmUmAmUmUmUfAmUfUmUfGmUmGmsUmsAm |
| TJR101188 | 331 | AmsCmsAmAmAmUmAfAfAfUmAmAmAmAmUmGmUmUmUm | 422 | AmsAfsAmCmAmUfUmUmUmAmUmUfUmAfUmUfUmGmUmsGmsUm |
| TJR101190 | 332 | AmsAmsAmUmAmAmAfUfAfAmAmAmUmGmUmUmUmAmUm | 423 | AmsUfsAmAmAmCfAmUmUmUmUmAfUmUfUmAfUmUmUmsGmsUm |
| TJR101191 | 333 | AmsAmsAmUmAmAmAfAfUfGmUmUmUmAmUmUmCmCmAm | 424 | UmsGfsGmAmAmUfAmAmAmCmAmUfUmUfUmAfUmUmUmsAmsUm |

In above Table 1B, the direction from the 5' end to the 3' end is directed by a sequence from the left to the right; the lowercase letter m indicates that the nucleoside adjacent to the left of the letter m is a 2'-methoxy-modified nucleoside; the lowercase letter f indicates that the nucleoside adjacent to the left of the letter f is a 2'-fluoro-modified nucleoside; the lowercase letter s indicates that linkage between two adjacent nucleosides flanking the letter s or between the nucleoside linked thereto and the delivery group I-14 is linkage by a phosphorothioate diester group; unless otherwise specified, two adjacent nucleosides are linked by a phosphodiester group; unless otherwise specified, the 3' position of the first nucleotide at the 3' end of each strand is hydroxy; the 5' position of the first nucleotide at the 5' end of each strand is hydroxy.

The structures of the 2'-methoxy-modified nucleoside, the 2'-fluoro-modified nucleoside, the phosphorothioate diester group, the phosphodiester group, and I-14 are shown in the table below. When the siRNAs or siRNA conjugates of the present disclosure are present in the form of a salt, e.g., in the form of a sodium salt, the structures in the form of a salt corresponding to the structures in Table 2 below are also within the claimed scope of the present disclosure.

Table 2

| Structure | Name |
|---|---|
| 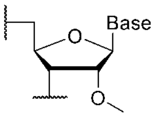 | 2'-Methoxy-modified nucleoside |
| 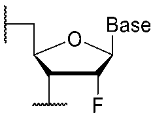 | 2'-Fluoro-modified nucleoside |
| 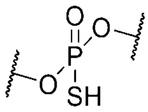 | Phosphorothioate diester group |
| 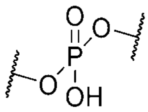 | Phosphodiester group |
| 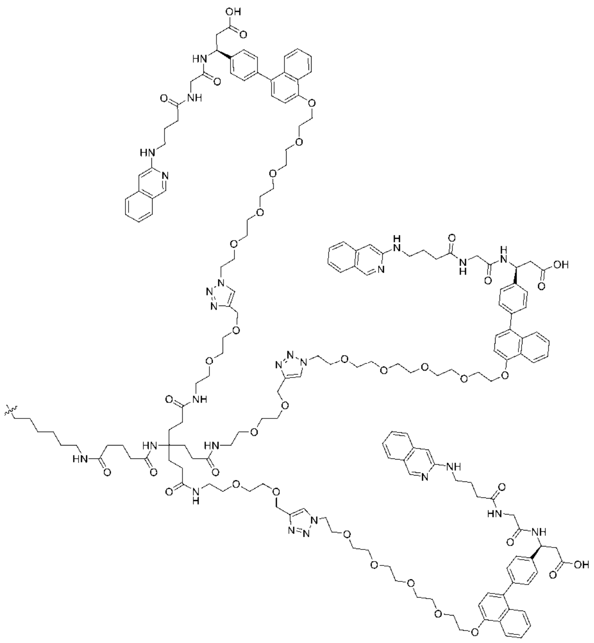 | I-14 |
| In those structures, Base represents a base. | |

**Example 2. Synthesis of siRNAs of Present Disclosure**

The siRNA synthesis followed the conventional phosphoramidite solid-phase synthesis method. The synthesis process is briefly described below: Nucleoside phosphoramidite monomers were linked one by one according to the synthesis program on a Dr. Oligo48 synthesizer (Biolytic), starting at a universal CPG support. The nucleoside monomer starting materials 2'-F RNA, 2'-O-methyl RNA, and other nucleoside phosphoramidite monomers were purchased from Shanghai Hongene Biotech Corporation or Suzhou GenePharma Co., Ltd. 5-Ethylthio-1H-tetrazole (ETT) was used as an activator (a 0.6 M solution in acetonitrile), a 0.22 M solution of PADS in acetonitrile and collidine (1:1 by volume) (Suzhou Kroma BioScience, Inc.) as a sulfurizing agent, and an iodopyridine/water solution (Suzhou Kroma BioScience, Inc.) as an oxidant.

After completion of solid-phase synthesis, oligoribonucleotides were cleaved from the solid support and soaked in a solution of 28% ammonia water and ethanol (3:1) at 50 °C for 16 h. Centrifugation was then performed, and the supernatant was transferred to another centrifuge tube. After the supernatant was concentrated to dryness by evaporation, the residue was purified by C18 reversed-phase chromatography with 0.1 M TEAA and acetonitrile as the mobile

phase, and DMTr was removed using a 3% trifluoroacetic acid solution. The target oligonucleotides were collected, then lyophilized, identified as the target products by LC-MS, and quantified by UV (260 nm) using nanodrop.

The resulting single-stranded oligonucleotides were complementarily paired in an equimolar ratio and annealed. The final double-stranded siRNA was dissolved in 1× PBS, and the solution was adjusted to the concentration required for the experiment and set aside for later use. The synthesis methods for oligonucleotides containing I-14 were as described in Example 3 and Example 4 below.

**Example 3. Synthesis of Ligand Compound I-12**

The synthesis scheme for compound **I-12** is as follows:

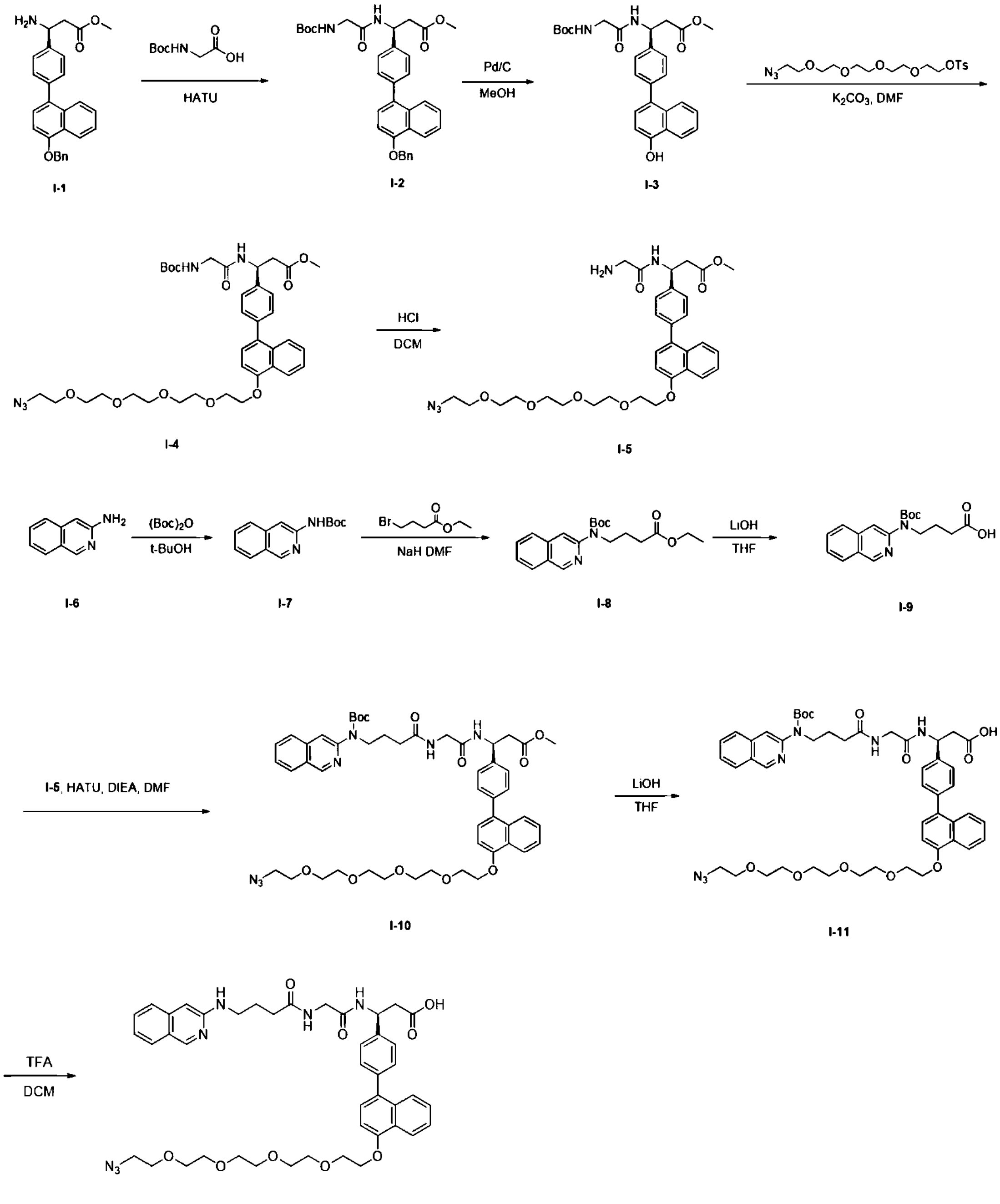


I-1 I-2 I-3 I-4 I-5 I-6 I-7 I-8 I-9 I-10 I-11

I-12

### 3-1 Synthesis of compound I-2

N,N-Diisopropylethylamine (132 mL, 799.2 mmol), O-(7-azabenzotriazol-1-yl)-N,N,N',N'-tetramethyluronium hexafluorophosphate (66.85 g, 175.8 mmol), and compound **I-1** (78.93 g, 191.8 mmol, prepared according to the method described in patent application WO2019089765A1) were added to a solution of N-(tert-butoxycarbonyl)glycine (28.00 g, 159.8 mmol, commercially available) in N-N-dimethylformamide (200 mL), and the mixture was purged 3 times with nitrogen. The reaction system was stirred at room temperature for 3 h under a nitrogen atmosphere until LCMS indicated the completion of the reaction. The reaction mixture was quenched with water and extracted with ethyl acetate. The combined organic phases were washed with saturated brine and dried over anhydrous sodium sulfate. The concentrated crude product was purified by a normal-phase silica gel column to give compound **I-2** (60.0 g, yield: 66%). LCMS: MS (ESI) m/z = 513.6 $[M+H-56]^+$.

### 3-2 Synthesis of compound I-3

10% palladium on carbon (0.23 g) was added to a solution of compound **I-2** (2.30 g, 4.05 mmol) in methanol (30.0 mL) under a hydrogen atmosphere. The reaction system was stirred at room temperature for 8 h until LCMS indicated the completion of the reaction. The reaction mixture was filtered and concentrated to give compound **I-3** (1.80 g, yield: 93%). $^1$H NMR (400 MHz, DMSO) $\delta$ 10.27 (s, 1H), 8.39 (d, $J$ = 8.4 Hz, 1H), 8.30-8.09 (m, 1H), 7.75 (dd, $J$ = 6.4, 3.2 Hz, 1H), 7.54-7.40 (m, 4H), 7.37 (d, $J$ = 8.4 Hz, 2H), 7.23 (d, $J$ = 7.6 Hz, 1H), 6.95 (dd, $J$ = 14.0, 6.8 Hz, 2H), 5.32 (q, $J$ = 7.6 Hz, 1H), 3.60 (s, 3H), 3.57 (d, $J$ = 6.0 Hz, 2H), 2.88 (t, $J$ = 6.0 Hz, 2H), 1.39 (s, 9H).

### 3-3 Synthesis of compound I-4

Azide-PEG5-Tos (1.74 g, 4.18 mmol) and potassium carbonate (0.58 g, 4.18 mmol) were added to a solution of compound **I-3** (1.00 g, 2.09 mmol) in N,N-dimethylformamide (10.0 mL). The reaction system was heated to 80 °C and stirred overnight until LCMS indicated the completion of the reaction. The reaction mixture was quenched with a saturated sodium bicarbonate solution and extracted with ethyl acetate (3 times, 40 mL each time). The combined organic phases were washed with saturated brine and dried over anhydrous sodium sulfate. The concentrated crude product was purified by a normal-phase silica gel column to give compound **I-4** (1.30, yield: 85%). LCMS: MS (ESI) m/z = 724.5 $[M+H]^+$.

### 3-4 Synthesis of compound I-5

A solution (20.0 mL) of hydrochloric acid in 1,4-dioxane was added to a solution of compound **I-4** (1.30 g, 1.79 mmol) in dichloromethane (20.0 mL). The reaction system was stirred at room temperature overnight until LCMS indicated the completion of the reaction. The reaction mixture was concentrated to give compound **I-5** (1.10 g, yield: 98%). LCMS: MS (ESI) m/z = 624.4 $[M+H]^+$.

### 3-5 Synthesis of compound I-7

Di-tert-butyl dicarbonate (6.58 g, 30.17 mmol) was added to a solution of compound **I-6** (2.90 g, 20.11 mmol, commercially available) in tert-butanol (50.0 mL). The reaction system was stirred at room temperature overnight until LCMS indicated the completion of the reaction. The reaction mixture was concentrated to give a crude product, which was then purified by a normal-phase silica gel column to give compound **I-7** (3.90 g, yield: 79%). LCMS: MS (ESI) m/z = 245.4 $[M+H]^+$.

### 3-6 Synthesis of compound I-8

Sodium hydride (0.19 g, 4.80 mmol) was added to a solution of compound **I-7** (0.98 g, 4.00 mmol) in N,N-dimethylformamide (10.0 mL) at 0 °C, and the mixture was stirred for 30 min with the temperature maintained. Then, ethyl 4-bromobutyrate (0.93 mL, 4.81 mmol) was added. The reaction system was stirred at room temperature overnight until LCMS indicated the completion of the reaction. The reaction mixture was quenched with water and extracted with ethyl acetate (3 times, 50 mL each time). The combined organic phases were washed with saturated brine and dried over anhydrous sodium sulfate. The concentrated crude product was purified by a normal-phase silica gel column to give compound **I-8** (1.30 g, yield: 91%). LCMS: MS (ESI) m/z =359.5 $[M+H]^+$.

### 3-7 Synthesis of compound I-9

A 1 N lithium hydroxide (20.0 mL) solution was added to a solution of compound **I-8** (1.30 g, 3.63 mmol) in

tetrahydrofuran (20.0 mL). The reaction system was stirred at room temperature for 2 h until LCMS indicated the completion of the reaction. The reaction mixture was adjusted to pH 2 with 1 N HCl and extracted with ethyl acetate (3 times, 20 mL each time). The combined organic phases were washed with saturated brine and dried over anhydrous sodium sulfate. The filtrate was concentrated to give compound **I-9** (1.18 g, yield: 98%). LCMS: MS (ESI) m/z =331.1 $[M+H]^+$.

### 3-8 Synthesis of compound I-10

N,N-Diisopropylethylamine (0.39 g, 3.03 mmol), O-(7-azabenzotriazol-1-yl)-N,N,N',N'-tetramethyluronium hexafluorophosphate (0.28 g, 0.73 mmol), and compound **I-5** (0.28 g, 0.72 mmol) were added to compound **I-9** (0.20 g, 0.61 mmol) in N-N-dimethylformamide (5.0 mL). The reaction system was stirred at room temperature for 2 h until LCMS indicated the completion of the reaction. The reaction mixture was quenched with water and extracted with ethyl acetate (3 times, 40 mL each time). The combined organic phases were washed with saturated brine and dried over anhydrous sodium sulfate. The concentrated crude product was purified by a normal-phase silica gel column to give compound **I-10** (0.30 g, yield: 53%). LCMS: MS (ESI) m/z = 936.7 $[M+H]^+$.

### 3-9 Synthesis of compound I-11

A 1 N lithium hydroxide (2.0 mL) solution was added to a solution of compound **I-10** (0.07 g, 0.08 mmol) in tetrahydrofuran (2.0 mL). The reaction system was stirred at room temperature for 2 h until LCMS indicated the completion of the reaction. The reaction mixture was adjusted to pH 2 with 1 N HCl and extracted with ethyl acetate (3 times, 20 mL each time). The combined organic phases were washed with saturated brine and dried over anhydrous sodium sulfate. The filtrate was concentrated to give compound **I-11** (0.27 g, yield: 91%). LCMS: MS (ESI) m/z =922.1 $[M+H]^+$.

### 3-10 Synthesis of compound I-12

Trifluoroacetic acid (4.0 mL) was added to a solution of compound **I-11** (0.27 g, 0.29 mmol) in dichloromethane (4.0 mL). The reaction system was stirred at room temperature for 3 h until LCMS indicated the completion of the reaction. The reaction mixture was concentrated to give a crude product, which was then purified by preparative high performance liquid chromatography to give compound **I-12** (0.09 g, yield: 37%). LCMS: MS (ESI) m/z =822.1 $[M+H]^+$. $^1H$ NMR (400 MHz, DMSO) δ 8.97 (s, 1H), 8.51 (d, *J* = 8.4 Hz, 1H), 8.33-8.23 (m, 1H), 8.15 (t, *J* = 5.6 Hz, 1H), 7.88 (d, *J* = 8.4 Hz, 1H), 7.79-7.72 (m, 1H), 7.63 (d, *J* = 8.4 Hz, 1H), 7.51 (m, 8H), 7.31 (d, *J* = 8.0 Hz, 1H), 7.26-7.20 (m, 1H), 7.04 (d, *J* = 8.0 Hz, 1H), 6.91 (s, 1H), 5.32 (q, *J* = 7.2 Hz, 1H), 4.34-4.31 (m, 2H), 3.95-3.91 (m, 2H), 3.78 (d, *J* = 5.6 Hz, 2H), 3.69 (dd, *J* = 6.0, 3.6 Hz, 2H), 3.61-3.50 (m, 13H), 3.38-3.34 (m, 2H), 3.29 (t, *J* = 7.2 Hz, 2H), 2.80 (d, *J* = 7.2 Hz, 2H), 2.29 (t, *J* = 7.2 Hz, 2H), 1.86 (p, *J* = 7.2 Hz, 2H).

The binding ability of compound I-12 to the αvβ6 protein was determined by the conventional fluorescence polarization (FP) method in the art, and the $IC_{50}$ value of the binding of compound I-12 to the human recombinant αvβ6 protein was determined to be 79.86 nM, indicating that compound I-12 is a ligand capable of binding to the αvβ6 protein. The specific assay method is as follows:

3.5 μL of test compound I-12 was added to a 384-well black plate. For the test compound, 12 concentration points were set up. The highest concentration was 10 μM, and 3-fold serial dilution was performed. DMSO control wells were used to determine the maximum signal, and 500 nM compound CWHM-12 (MCE, HY-18644) control wells were used as the minimum signal. Then, 3.5 μL of 4× human recombinant αvβ6 protein working solution was added. After incubation at room temperature for 15 min, 7 μL of 2× fluorescent RGD peptide solution was added to each well. The plate was incubated at room temperature for 1 h, and then FP signals were read using Envision. Using GraphPad Prism, the inhibition curve fitting was performed, and the $IC_{50}$ value was calculated. The $IC_{50}$ value (Z prime > 0.5) was calculated using the following formula:

$$\text{Curve fitting formula: } Y = \text{Bottom} + (\text{Top - Bottom})/(1 + 10\text{^}((\text{LogIC50 - X}) \times \text{Hill Slope}))$$

X: Log inhibitor concentration; Y: inhibition rate %.

### Example 4. Synthesis of siRNA Conjugates of Present Disclosure

**4-1 Synthesis of sense strands linked to I-13'**

The sense strands containing I-13' in Table 3 were synthesized according to the same method as that in Example 2, the only difference being that at the position corresponding to I-13', the alkynyl-containing phosphoramidite monomer I-13, prepared according to the method described in the patent application WO2019161213A1, was used in the solid-phase synthesis to obtain the sense strands containing I-13'. The structures of I-13 and I-13' are shown below:

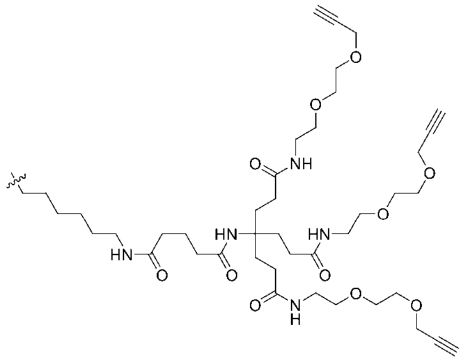

I-13'

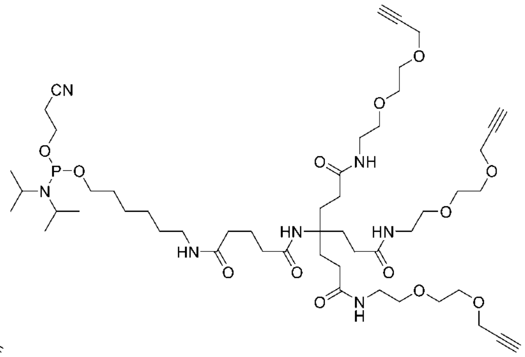

I-13

Table 3. Sequences of sense strands linked to I-13' or I-14

| No. | SS strand (5'-3') | SEQ ID NO |
|---|---|---|
| I-13-28 | I-**13**'UmsGmsGmGmUmAmGfAfUfGmUmCmAmAmUmAmAmAmUm | **425** |
| I-13-29 | **I-13**'UmsGmsAmCmUmCmAfGfAfAmAmCmAmAmAmAmAmAmUm | **426** |
| I-13-30 | **I-13'** AmsUmsUmCmAmAmGfAfAfAmGmAmAmAmUmAmGmAmAm | **427** |
|  | **I-14**UmsGmsGmGmUmAmGfAfUfGmUmCmAmAmUmAmAmAmUm | **28** |
|  | **I-14**UmsGmsAmCmUmCmAfGfAfAmAmCmAmAmAmAmAmAmUm | **29** |
|  | **I-14**AmsUmsUmCmAmAmGfAfAfAmGmAmAmAmUmAmGmAmAm | **30** |

**4-2 Synthesis of sense strands containing I-14** Stock solutions of 0.5 M tris(3-hydroxypropyltriazolylmethyl) amine (THPTA), 0.5 M Cu(II) sulfate pentahydrate (Cu(II)$SO_4 \cdot 5H_2O$), and 2 M sodium ascorbate solutions were prepared in deionized water. In addition, a 2 M TEAA solution (10000 ng/μL) of I-13-28 (2.5 μmol) prepared in step 4-1 and a 25 mmol/L DMSO solution of I-12 (35 μmol) were prepared. The DMSO solution containing I-12 was added to the 2 M TEAA solution containing I-13-28, and the mixture was shaken and mixed well. Then 75 μL of 0.5 M Cu(II) sulfate pentahydrate (Cu(II)$SO_4$ $5H_2O$), 75 μL of 0.5 M THPTA, and 18 μL of 2 M ascorbate were sequentially added to a centrifuge tube. After 1 h of shaking in a thermostatic reactor, the reaction was completed as detected by LCMS. The mixture was centrifuged, and the supernatant was then taken and purified by hydrophobic chromatography (A: ammonium sulfate buffer, B: purified water) to give the sense strand containing I-14 set forth in SEQ ID NO: 28.

The sense strands set forth in SEQ ID NO: 29 and SEQ ID NO: 30 were prepared using the same method as described above, the only difference being that I-13-28 in the previous steps was replaced with 1-13-29 and 1-13-30, respectively.

**4-3 Synthesis of siRNA conjugates containing I-14**

According to the same method as that in Example 2, the sense strands containing I-14 obtained in step 4-2 and the corresponding antisense strands were paired in an equimolar ratio in a complementary manner and annealed to obtain siRNA conjugates containing I-14.

**Example 5. On-Target Activity of siRNAs at 3 Concentration Points Using psiCHECK**

The siRNAs of the present disclosure were subjected to an *in vitro* molecular-level simulation of on-target activity screening in HEK293A cells using 3 concentration gradients (10 nM, 1 nM, and 0.1 nM).

HEK293A cells were cultured at 37 °C with 5% $CO_2$ in a DMEM high glucose culture medium containing 10% fetal

bovine serum. 24 h prior to transfection, the HEK293A cells were seeded into a 96-well plate at a density of $8 \times 10^3$ cells per well, with each well containing 100 μL of the culture medium.

The cells were co-transfected with the siRNAs and the corresponding MMP7 full-length plasmids using Lipofectamine2000 (ThermoFisher, 11668019) according to the instructions. 0.3 μL of Lipofectamine2000 was used for each well. The amount of plasmids for transfection was 40 ng per well. For the on-target sequence plasmids, a total of 3 concentration points were set up for the siRNAs, i.e., 10 nM, 1 nM, and 0.1 nM, with duplicate wells for each concentration. 24 h after transfection, the on-target levels were determined using a Dual-Luciferase Reporter Assay System (Promega, E2940). The activity results are recorded in Table 4. As shown in Table 4, the siRNAs of the present disclosure have good MMP7 mRNA inhibitory activity in the psi-CHECK system.

Table 4. Activity results of siRNAs at 3 concentration points in the psi-Check system

| Double strand No. | 10 nM remaining expression (%) | STDEV (%) | 1 nM remaining expression (%) | STDEV (%) | 0.1 nM remaining expression (%) | STDEV (%) |
|---|---|---|---|---|---|---|
| TJR101103 | 6.09 | 0.52 | 7.25 | 0.25 | 22.53 | 0.32 |
| TJR101182 | 7.35 | 0.11 | 9.05 | 0.78 | 24.61 | 0.68 |
| TJR101129 | 7.12 | 0.01 | 7.95 | 0.21 | 25.33 | 2.11 |
| TJR101186 | 5.19 | 0.19 | 7.56 | 0.03 | 27.75 | 1.70 |
| TJR101119 | 3.08 | 0.03 | 6.19 | 2.28 | 29.49 | 0.83 |
| TJR101160 | 4.56 | 0.52 | 9.25 | 0.18 | 32.48 | 0.86 |
| TJR101169 | 5.88 | 0.06 | 9.25 | 0.18 | 32.48 | 0.86 |
| TJR101181 | 10.92 | 0.03 | 13.87 | 0.38 | 33.04 | 0.12 |
| TJR101179 | 8.68 | 0.59 | 10.89 | 0.02 | 33.86 | 1.06 |
| TJR101188 | 9.04 | 0.14 | 18.11 | 0.29 | 33.95 | 0.70 |
| TJR101184 | 8.92 | 0.07 | 10.48 | 0.25 | 34.58 | 0.31 |
| TJR101102 | 9.01 | 0.73 | 13.11 | 1.26 | 37.41 | 1.64 |
| TJR101108 | 8.17 | 0.21 | 9.78 | 0.45 | 38.84 | 0.34 |
| TJR101104 | 6.65 | 0.52 | 10.17 | 0.04 | 39.72 | 2.03 |
| TJR101128 | 4.27 | 0.80 | 9.29 | 3.83 | 40.11 | 3.32 |
| TJR101183 | 7.12 | 0.28 | 11.18 | 0.09 | 40.14 | 3.64 |
| TJR101174 | 4.26 | 0.32 | 13.54 | 0.59 | 41.07 | 2.82 |
| TJR101161 | 6.92 | 0.25 | 12.82 | 0.53 | 41.31 | 0.89 |
| TJR101170 | 7.31 | 0.09 | 12.82 | 0.53 | 41.31 | 0.89 |
| TJR101151 | 7.26 | 1.00 | 11.58 | 0.12 | 42.79 | 1.32 |
| TJR101173 | 6.15 | 0.51 | 12.53 | 0.16 | 43.85 | 0.27 |
| TJR101112 | 9.25 | 0.15 | 13.60 | 0.05 | 44.97 | 0.77 |
| TJR101189 | 9.19 | 0.47 | 17.95 | 0.68 | 46.17 | 1.10 |
| TJR101180 | 7.35 | 0.51 | 14.99 | 1.37 | 46.47 | 2.61 |
| TJR101120 | 3.30 | 0.40 | 10.50 | 3.07 | 48.02 | 1.40 |
| TJR101131 | 9.74 | 0.18 | 14.53 | 0.26 | 49.14 | 0.81 |
| TJR101159 | 5.42 | 0.49 | 13.37 | 1.17 | 49.35 | 0.83 |
| TJR101168 | 7.43 | 0.41 | 13.37 | 1.17 | 49.35 | 0.83 |
| TJR101109 | 10.35 | 1.08 | 14.03 | 0.91 | 50.44 | 1.87 |
| TJR101105 | 6.97 | 0.75 | 12.51 | 0.34 | 51.22 | 0.83 |
| TJR101185 | 7.81 | 0.06 | 14.30 | 0.04 | 51.47 | 0.92 |

(continued)

| Double strand No. | 10 nM remaining expression (%) | STDEV (%) | 1 nM remaining expression (%) | STDEV (%) | 0.1 nM remaining expression (%) | STDEV (%) |
|---|---|---|---|---|---|---|
| TJR101178 | 6.17 | 0.21 | 15.38 | 0.03 | 51.65 | 0.17 |
| TJR101107 | 8.10 | 0.34 | 12.25 | 1.04 | 51.72 | 5.61 |
| TJR101127 | 4.70 | 0.66 | 11.34 | 4.21 | 52.56 | 2.96 |
| TJR101148 | 13.89 | 1.23 | 20.76 | 0.28 | 54.88 | 1.87 |
| TJR101171 | 4.83 | 0.18 | 15.98 | 1.17 | 55.81 | 4.36 |
| TJR101101 | 10.68 | 0.97 | 19.17 | 0.41 | 55.99 | 0.26 |
| TJR101121 | 5.04 | 0.97 | 13.59 | 6.38 | 56.37 | 1.13 |
| TJR101114 | 7.18 | 0.37 | 12.92 | 0.54 | 56.48 | 0.17 |
| TJR101150 | 9.46 | 0.22 | 18.99 | 0.11 | 57.22 | 3.99 |
| TJR101095 | 5.80 | 0.39 | 12.35 | 1.07 | 57.67 | 0.89 |
| TJR101142 | 9.91 | 0.79 | 16.67 | 0.07 | 58.47 | 5.52 |
| TJR101096 | 5.99 | 0.11 | 15.11 | 0.95 | 59.47 | 5.52 |
| TJR101111 | 11.01 | 0.17 | 17.87 | 0.54 | 60.45 | 6.02 |
| TJR101130 | 7.43 | 0.26 | 15.85 | 0.56 | 61.16 | 0.69 |
| TJR101154 | 7.03 | 0.62 | 17.16 | 1.31 | 62.55 | 2.38 |
| TJR101163 | 7.19 | 0.39 | 17.16 | 1.31 | 62.55 | 2.38 |
| TJR101136 | 10.88 | 0.67 | 20.61 | 0.54 | 62.92 | 0.32 |
| TJR101190 | 11.83 | 0.36 | 26.04 | 0.60 | 63.00 | 0.22 |
| TJR101099 | 10.78 | 0.02 | 21.58 | 0.12 | 63.59 | 11.60 |
| TJR101158 | 7.81 | 0.62 | 21.07 | 0.10 | 66.06 | 0.93 |
| TJR101167 | 6.22 | 0.13 | 21.07 | 0.10 | 66.06 | 0.93 |
| TJR101126 | 4.29 | 0.54 | 16.62 | 6.71 | 67.16 | 2.42 |
| TJR101145 | 15.30 | 0.16 | 25.46 | 0.68 | 67.51 | 2.34 |
| TJR101149 | 9.57 | 0.54 | 22.55 | 1.05 | 68.81 | 2.42 |
| TJR101093 | 12.46 | 0.31 | 21.59 | 0.70 | 68.96 | 2.58 |
| TJR101146 | 12.68 | 0.30 | 24.00 | 1.14 | 69.16 | 2.07 |
| TJR101092 | 14.64 | 0.21 | 26.71 | 1.25 | 70.17 | 6.97 |
| TJR101155 | 8.75 | 0.28 | 21.49 | 0.09 | 70.41 | 1.76 |
| TJR101164 | 7.53 | 0.52 | 21.49 | 0.09 | 70.41 | 1.76 |
| TJR101175 | 7.06 | 0.19 | 24.78 | 1.48 | 70.72 | 2.56 |
| TJR101097 | 34.69 | 0.93 | 83.54 | 1.03 | 71.56 | 33.11 |
| TJR101152 | 10.44 | 3.91 | 21.57 | 0.09 | 72.15 | 1.73 |
| TJR101139 | 14.17 | 1.19 | 25.32 | 1.81 | 72.65 | 3.17 |
| TJR101187 | 9.62 | 0.41 | 29.63 | 1.24 | 72.98 | 1.81 |
| TJR101094 | 20.87 | 0.55 | 29.33 | 0.70 | 74.67 | 2.24 |
| TJR101157 | 8.16 | 1.00 | 31.56 | 0.95 | 76.69 | 2.57 |
| TJR101166 | 7.64 | 0.23 | 31.56 | 0.95 | 76.69 | 2.57 |
| TJR101153 | 9.61 | 0.45 | 23.41 | 0.30 | 76.89 | 1.20 |

(continued)

| Double strand No. | 10 nM remaining expression (%) | STDEV (%) | 1 nM remaining expression (%) | STDEV (%) | 0.1 nM remaining expression (%) | STDEV (%) |
|---|---|---|---|---|---|---|
| TJR101162 | 8.28 | 0.70 | 23.41 | 0.30 | 76.89 | 1.20 |
| TJR101098 | 14.10 | 0.18 | 18.85 | 0.42 | 77.14 | 22.28 |
| TJR101156 | 12.77 | 0.24 | 35.32 | 3.55 | 77.28 | 4.23 |
| TJR101165 | 7.07 | 0.05 | 35.32 | 3.55 | 77.28 | 4.23 |
| TJR101124 | 8.68 | 0.81 | 28.89 | 11.27 | 79.07 | 1.55 |
| TJR101177 | 12.53 | 1.15 | 35.12 | 0.43 | 79.71 | 0.65 |
| TJR101191 | 11.29 | 1.26 | 40.08 | 0.99 | 79.73 | 0.43 |
| TJR101138 | 20.16 | 1.17 | 40.01 | 1.79 | 81.89 | 1.93 |
| TJR101106 | 8.62 | 0.81 | 31.51 | 0.60 | 82.82 | 2.86 |
| TJR101141 | 11.96 | 0.23 | 35.51 | 1.37 | 82.91 | 6.22 |
| TJR101144 | 31.04 | 0.93 | 48.40 | 0.48 | 85.87 | 2.80 |
| TJR101113 | 7.55 | 0.07 | 26.30 | 2.88 | 87.15 | 2.10 |
| TJR101140 | 18.71 | 1.27 | 39.52 | 2.34 | 88.75 | 3.87 |
| TJR101137 | 11.01 | 0.95 | 40.11 | 0.66 | 89.46 | 3.04 |
| TJR101091 | 37.54 | 1.03 | 80.88 | 5.14 | 89.63 | 4.65 |
| TJR101115 | 11.32 | 0.70 | 34.98 | 0.67 | 90.09 | 1.69 |
| TJR101147 | 19.05 | 0.28 | 42.03 | 1.54 | 90.25 | 0.86 |
| TJR101176 | 13.99 | 1.01 | 41.47 | 3.65 | 93.50 | 1.26 |
| TJR101133 | 18.00 | 0.72 | 54.84 | 3.67 | 94.52 | 0.34 |
| TJR101143 | 44.99 | 0.34 | 75.87 | 1.35 | 94.75 | 8.93 |
| TJR101122 | 14.84 | 1.79 | 54.99 | 21.36 | 95.35 | 4.47 |
| TJR101116 | 11.33 | 2.25 | 48.44 | 0.49 | 96.93 | 6.11 |
| TJR101110 | 20.92 | 0.24 | 67.50 | 0.98 | 98.16 | 0.75 |
| TJR101125 | 61.83 | 7.20 | 87.15 | 28.36 | 98.81 | 3.77 |
| TJR101117 | 16.49 | 0.45 | 53.78 | 2.57 | 98.94 | 1.46 |
| TJR101118 | 15.02 | 1.19 | 56.58 | 1.99 | 100.05 | 0.61 |

**Example 6. On-Target Activity of siRNAs at 9 Concentration Points Using psiCHECK**

The siRNAs of the present disclosure were subjected to an *in vitro* molecular-level simulation of on-target activity screening in HEK293A cells using 9 concentration gradients.

HEK293A cells were cultured at 37 °C with 5% $CO_2$ in a DMEM high glucose culture medium containing 10% fetal bovine serum. 24 h prior to transfection, the HEK293A cells were seeded into a 96-well plate at a density of $1 \times 10^4$ cells per well, with each well containing 100 μL of the culture medium.

The cells were co-transfected with the siRNAs and the corresponding plasmids using Lipofectamine2000 (ThermoFisher, 11668019) according to the instructions. 0.3 μL of Lipofectamine2000 was used for each well. The amount of plasmids for transfection was 20 ng per well. For the on-target sequence plasmids, a total of 9 concentration points were set up for the siRNAs, with the highest concentration point final concentration being 20 nM. A 3-fold serial dilution was performed, resulting in 20.0000 nM, 6.6667 nM, 2.2222 nM, 0.7407 nM, 0.2469 nM, 0.0823 nM, 0.0274 nM, 0.0091 nM, and 0.0030 nM. 24 h after transfection, the on-target levels were determined using a Dual-Luciferase Reporter Assay System (Promega, E2940). The results are shown in Table 5. As shown in Table 5, the siRNAs of the present disclosure all have good MMP7 mRNA on-target activity at the 9 concentration points in the psi-CHECK system.

Table 5. Activity results of siRNAs at 9 concentration points in the psiCHECK system

| Remaining percentage of target gene mRNA expression in psi-check (mean) | | | | | |
|---|---|---|---|---|---|
| Double strand code | 20.0000 nM | 6.6667 nM | 2.2222 nM | 0.7407 nM | 0.2469 nM |
| TJR101100 | 13.32 | 12.89 | 13.72 | 16.55 | 28.33 |
| TJR101102 | 7.64 | 7.25 | 8.29 | 9.24 | 12.60 |
| TJR101103 | 9.76 | 7.12 | 7.21 | 7.35 | 9.30 |
| TJR101104 | 6.36 | 6.46 | 7.09 | 8.73 | 14.90 |
| TJR101108 | 9.18 | 8.38 | 7.85 | 8.33 | 10.77 |
| TJR101112 | 11.38 | 8.56 | 9.87 | 11.74 | 15.55 |
| TJR101119 | 6.46 | 6.82 | 7.37 | 8.33 | 11.93 |
| TJR101120 | 5.49 | 4.93 | 5.79 | 7.57 | 12.64 |
| TJR101123 | 6.57 | 8.44 | 10.96 | 15.59 | 31.46 |
| TJR101128 | 8.06 | 7.44 | 8.23 | 9.73 | 15.02 |
| TJR101129 | 8.97 | 6.27 | 6.35 | 7.09 | 9.20 |
| TJR101131 | 8.95 | 8.98 | 10.28 | 11.53 | 17.68 |
| TJR101134 | 13.29 | 12.50 | 14.83 | 19.86 | 32.89 |
| TJR101135 | 12.23 | 9.47 | 9.28 | 10.82 | 18.39 |
| TJR101151 | 7.06 | 7.04 | 8.14 | 9.37 | 13.25 |
| TJR101159 | 5.20 | 4.75 | 5.19 | 6.21 | 9.13 |
| TJR101160 | 3.84 | 3.97 | 4.54 | 5.46 | 7.68 |
| TJR101161 | 8.21 | 7.39 | 7.65 | 8.59 | 11.02 |
| TJR101168 | 6.06 | 5.49 | 5.80 | 8.40 | 17.00 |
| TJR101169 | 7.09 | 6.38 | 6.43 | 6.89 | 10.06 |
| TJR101170 | 6.82 | 7.34 | 7.81 | 9.32 | 14.66 |
| TJR101172 | 4.11 | 4.92 | 5.81 | 7.43 | 13.15 |
| TJR101173 | 5.14 | 5.18 | 5.77 | 6.82 | 9.82 |
| TJR101174 | 3.07 | 3.03 | 4.56 | 6.65 | 10.97 |
| TJR101179 | 11.28 | 8.23 | 7.45 | 7.93 | 10.23 |
| TJR101180 | 6.03 | 5.39 | 5.37 | 6.93 | 10.66 |
| TJR101181 | 9.84 | 10.23 | 10.91 | 11.46 | 12.41 |
| TJR101182 | 7.28 | 7.40 | 7.76 | 8.40 | 9.30 |
| TJR101183 | 5.86 | 5.65 | 5.94 | 6.78 | 9.16 |
| TJR101184 | 8.85 | 7.23 | 7.46 | 6.92 | 6.94 |
| TJR101186 | 6.38 | 4.14 | 4.86 | 5.43 | 5.95 |
| TJR101188 | 8.97 | 8.29 | 9.03 | 10.99 | 14.70 |
| TJR101189 | 10.21 | 8.81 | 9.38 | 12.04 | 17.06 |
| Double strand code | 0.0823 nM | 0.0274 nM | 0.0091 nM | 0.0030 nM | $IC_{50}$ (nM) |
| TJR101100 | 49.52 | 78.03 | 98.18 | 107.34 | 0.081 |
| TJR101102 | 20.80 | 38.69 | 54.73 | 75.87 | 0.013 |
| TJR101103 | 14.03 | 29.47 | 48.50 | 76.42 | 0.009 |
| TJR101104 | 26.68 | 56.90 | 78.94 | 97.13 | 0.032 |

(continued)

| Remaining percentage of target gene mRNA expression in psi-check (mean) | | | | | |
|---|---|---|---|---|---|
| TJR101108 | 22.65 | 40.60 | 69.30 | 82.02 | 0.020 |
| TJR101112 | 31.00 | 50.65 | 82.92 | 92.66 | 0.032 |
| TJR101119 | 22.82 | 42.59 | 70.00 | 90.79 | 0.020 |
| TJR101120 | 26.56 | 45.22 | 69.46 | 82.89 | 0.023 |
| TJR101123 | 51.42 | 74.47 | 75.51 | 83.60 | 0.088 |
| TJR101128 | 30.38 | 57.56 | 79.37 | 96.60 | 0.035 |
| TJR101129 | 12.86 | 27.75 | 46.39 | 64.95 | 0.008 |
| TJR101131 | 27.00 | 49.84 | 72.92 | 88.25 | 0.027 |
| TJR101134 | 58.71 | 85.34 | 98.79 | 104.04 | 0.115 |
| TJR101135 | 35.54 | 56.65 | 71.89 | 80.06 | 0.039 |
| TJR101151 | 21.68 | 42.08 | 70.65 | 89.67 | 0.020 |
| TJR101159 | 16.71 | 35.02 | 55.74 | 68.26 | 0.013 |
| TJR101160 | 12.85 | 24.89 | 48.27 | 69.99 | 0.008 |
| TJR101161 | 19.06 | 38.94 | 67.73 | 84.10 | 0.018 |
| TJR101168 | 29.11 | 56.37 | 73.77 | 81.50 | 0.035 |
| TJR101169 | 17.75 | 35.60 | 67.04 | 82.23 | 0.017 |
| TJR101170 | 30.74 | 59.43 | 84.96 | 97.92 | 0.038 |
| TJR101172 | 28.88 | 55.54 | 77.01 | 90.58 | 0.033 |
| TJR101173 | 20.39 | 39.79 | 63.49 | 82.55 | 0.017 |
| TJR101174 | 22.81 | 44.36 | 70.53 | 91.17 | 0.022 |
| TJR101179 | 15.90 | 31.78 | 64.28 | 86.30 | 0.015 |
| TJR101180 | 22.41 | 39.29 | 57.31 | 73.62 | 0.015 |
| TJR101181 | 15.68 | 29.05 | 58.30 | 79.39 | 0.012 |
| TJR101182 | 13.04 | 21.96 | 50.94 | 82.26 | 0.009 |
| TJR101183 | 14.56 | 34.58 | 51.63 | 73.17 | 0.011 |
| TJR101184 | 8.48 | 14.09 | 27.04 | 52.06 | 0.003 |
| TJR101186 | 7.12 | 11.54 | 19.74 | 41.81 | 0.002 |
| TJR101188 | 22.99 | 44.14 | 70.68 | 90.39 | 0.021 |
| TJR101189 | 31.80 | 56.59 | 86.09 | 108.90 | 0.035 |

**Example 7. On-Target Activity of siRNAs at 7 Endogenous Concentration Points**

The siRNAs were subjected to an *in vitro* molecular-level activity screening in HCC38 cells using 7 concentration gradients.

HCC38 cells were cultured at 37 °C with 5% $CO_2$ in a 1640 culture medium containing 10% fetal bovine serum. 24 h prior to transfection, the HCC38 cells were seeded into a 96-well plate at a density of $1.5 \times 10^4$ cells per well, with each well containing 100 μL of the culture medium.

The cells were transfected with the siRNAs using Lipofectamine RNAi Max (Invitrogen, 13778-150) according to the instructions. 0.3 μL of Lipofectamine RNAi Max was used for each well. A total of 7 concentration points were set up for the siRNAs, i.e., 20.0000 nM, 10.0000 nM, 2.0000 nM, 0.4000 nM, 0.0800 nM, 0.0160 nM, and 0.0032 nM, respectively. 24 h after transfection, RNA was extracted for Q-PCR to detect the activity of siRNAs. The results are shown in Table 6. The siRNAs of the present disclosure exhibit good MMP7 mRNA on-target activity in HCC38 cells.

Table 6. Activity results of siRNAs at 7 concentration points in HCC38 cells

| Remaining percentage of target gene mRNA expression in HCC38 (mean) | | | | | |
|---|---|---|---|---|---|
| Double strand code | 20.0000 nM | 10.0000 nM | 2. 0000 nM | 0.4000 nM | 0.0800 nM |
| TJR101103 | 12.70 | 7.90 | 6.68 | 9.25 | 16.98 |
| TJR101104 | 13.07 | 7.14 | 7.01 | 8.77 | 13.68 |
| TJR101108 | 13.28 | 9.52 | 7.71 | 11.70 | 19.09 |
| TJR101112 | 14.86 | 10.60 | 8.30 | 10.76 | 17.69 |
| TJR101120 | 9.77 | 8.02 | 10.05 | 10.65 | 20.87 |
| TJR101183 | 13.43 | 14.31 | 16.85 | 21.98 | 26.03 |
| TJR101180 | 12.03 | 11.89 | 15.33 | 18.26 | 30.13 |
| TJR101181 | 9.96 | 13.89 | 12.34 | 17.89 | 20.25 |
| TJR101131 | 8.30 | 9.48 | 12.58 | 16.00 | 22.10 |
| TJR101134 | 7.39 | 10.27 | 10.95 | 27.91 | 63.80 |
| TJR101184 | 16.36 | 16.10 | 17.64 | 19.65 | 24.41 |
| TJR101135 | 7.92 | 7.42 | 10.59 | 11.96 | 22.72 |
| TJR101172 | 6.48 | 6.51 | 8.04 | 9.85 | 22.71 |
| TJR101188 | 12.04 | 13.97 | 17.67 | 21.07 | 37.88 |
| TJR101189 | 9.56 | 10.18 | 12.44 | 16.10 | 19.12 |
| TJR101129 | 9.95 | 12.22 | 12.41 | 21.52 | 41.46 |
| TJR101128 | 14.64 | 14.97 | 16.32 | 23.11 | 36.75 |
| TJR101159 | 42.67 | 40.80 | 41.11 | 45.03 | 45.99 |
| TJR101174 | 45.39 | 46.53 | 43.50 | 47.48 | 53.43 |
| TJR101179 | 14.77 | 13.86 | 13.45 | 19.27 | 21.69 |
| TJR101186 | 14.95 | 17.36 | 18.50 | 51.86 | 84.17 |
| Double strand code | 0.0160 nM | 0.0032 nM | $IC_{50}$ (nM) | | |
| TJR101103 | 38.10 | 79.80 | 0.010 | | |
| TJR101104 | 35.00 | 74.35 | 0.009 | | |
| TJR101108 | 55.70 | 83.05 | 0.020 | | |
| TJR101112 | 44.39 | 75.12 | 0.013 | | |
| TJR101120 | 55.33 | 79.54 | 0.020 | | |
| TJR101183 | 43.01 | 75.01 | 0.010 | | |
| TJR101180 | 63.29 | 91.71 | 0.028 | | |
| TJR101181 | 35.36 | 57.64 | 0.005 | | |
| TJR101131 | 50.88 | 76.96 | 0.016 | | |
| TJR101134 | 73.27 | 1850.20 | 0.143 | | |
| TJR101184 | 43.33 | 78.82 | 0.011 | | |
| TJR101135 | 55.28 | 85.12 | 0.020 | | |
| TJR101172 | 44.76 | 224.71 | 0.012 | | |
| TJR101188 | 71.61 | 87.76 | 0.046 | | |
| TJR101189 | 35.82 | 82.43 | 0.008 | | |
| TJR101129 | 85.35 | 94.79 | 0.063 | | |

(continued)

| Double strand code | 0.0160 nM | 0.0032 nM | $IC_{50}$ (nM) |
|---|---|---|---|
| TJR101128 | 68.75 | 102.22 | 0.038 |
| TJR101159 | 65.90 | 92.34 | 0.055 |
| TJR101174 | 67.29 | 89.56 | 0.152 |
| TJR101179 | 40.98 | 74.84 | 0.010 |
| TJR101186 | 100.33 | 108.03 | 0.385 |

**Example 8. On-Target Activity of siRNAs at 7 Endogenous Concentration Points**

The siRNAs were subjected to an *in vitro* molecular-level activity screening in HCC38 cells using 7 concentration gradients.

HCC38 cells were cultured at 37 °C with 5% $CO_2$ in a 1640 culture medium containing 10% fetal bovine serum. 24 h prior to transfection, the HCC38 cells were seeded into a 96-well plate at a density of $1.5 \times 10^4$ cells per well, with each well containing 100 μL of the culture medium.

The cells were transfected with the siRNAs using Lipofectamine RNAi Max (Invitrogen, 13778-150) according to the instructions. 0.3 μL of Lipofectamine RNAi Max was used for each well. A total of 7 concentration points were set up for the siRNAs, i.e., 20.0000 nM, 4.0000 nM, 0.8000 nM, 0.1600 nM, 0.0320 nM, 0.0064 nM, and 0.0013 nM, respectively. 24 h after transfection, RNA was extracted for Q-PCR to detect the activity of siRNAs. The results are shown in Table 7. The siRNAs of the present disclosure exhibit good MMP7 mRNA on-target activity in HCC38 cells.

Table 7. Activity results of siRNAs at 7 concentration points at the HCC38 cellular level

| Remaining percentage of target gene mRNA expression in HCC38 (mean) | | | | | |
|---|---|---|---|---|---|
| Double strand code | 20.0000 nM | 4.0000 nM | 0.8000 nM | 0.1600 nM | 0.0320 nM |
| TJR101182 | 6.00 | 8.15 | 8.74 | 10.53 | 14.40 |
| TJR101151 | 6.71 | 6.86 | 9.65 | 18.12 | 44.53 |
| TJR101100 | 4.91 | 7.46 | 9.42 | 20.62 | 61.70 |
| TJR101123 | 3.86 | 5.20 | 9.54 | 20.59 | 61.34 |
| TJR101168 | 6.70 | 8.84 | 11.60 | 25.67 | 62.10 |
| TJR101160 | 6.84 | 9.28 | 9.56 | 14.24 | 35.56 |
| TJR101173 | 12.78 | 13.18 | 22.55 | 26.34 | 64.09 |
| TJR101170 | 10.26 | 18.68 | 17.62 | 34.12 | 63.48 |
| TJR101161 | 12.02 | 10.41 | 15.82 | 33.31 | 65.27 |
| TJR101169 | 11.68 | 12.96 | 21.76 | 32.72 | 64.26 |
| TJR101119 | 12.90 | 23.44 | 37.72 | 47.61 | 64.88 |
| TJR101102 | 7.70 | 18.29 | 36.44 | 45.77 | 68.62 |
| Double strand code | 0.0064 nM | 0.0013 nM | $IC_{50}$ (nM) | | |
| TJR101182 | 30.11 | 69.85 | 0.006 | | |
| TJR101151 | 74.44 | 93.27 | 0.058 | | |
| TJR101100 | 96.70 | 99.21 | 0.119 | | |
| TJR101123 | 91.50 | 89.95 | 0.122 | | |
| TJR101168 | 89.04 | 100.78 | 0.127 | | |
| TJR101160 | 60.65 | 82.45 | 0.031 | | |
| TJR101173 | 88.05 | 94.21 | 0.055 | | |
| TJR101170 | 90.08 | 93.15 | 0.065 | | |

(continued)

| Double strand code | 0.0064 nM | 0.0013 nM | $IC_{50}$ (nM) | |
|---|---|---|---|---|
| TJR101161 | 78.15 | 89.38 | 0.066 | |
| TJR101169 | 99.74 | 92.30 | 0.066 | |
| TJR101119 | 89.67 | 93.47 | 0.119 | |
| TJR101102 | 97.04 | 96.73 | 0.113 | |

**Example 9. On-Target Activity of siRNAs at 9 Concentration Points Using psiCHECK**

The siRNAs were subjected to an *in vitro* molecular-level simulation of on-target activity screening in HEK293A cells using 9 concentration gradients.

HEK293A cells were cultured at 37 °C with 5% $CO_2$ in a DMEM high glucose culture medium containing 10% fetal bovine serum. 24 h prior to transfection, the HEK293A cells were seeded into a 96-well plate at a density of $1 \times 10^4$ cells per well, with each well containing 100 μL of the culture medium.

The cells were co-transfected with the siRNAs and the corresponding plasmids using Lipofectamine2000 (ThermoFisher, 11668019) according to the instructions. 0.3 μL of Lipofectamine2000 was used for each well. The amount of plasmids for transfection was 20 ng per well. For the on-target sequence plasmids, a total of 9 concentration points were set up for the siRNAs, with the highest concentration point final concentration being 20 nM. A 3-fold serial dilution was performed, resulting in 20.0000 nM, 6.6667 nM, 2.2222 nM, 0.7407 nM, 0.2469 nM, 0.0823 nM, 0.0274 nM, 0.0091 nM, and 0.0030 nM. 24 h after transfection, the on-target levels were determined using a Dual-Luciferase Reporter Assay System (Promega, E2940). The results are shown in Table 8. NA indicates no detection. As shown in Table 8, the siRNAs of the present disclosure exhibit good MMP7 mRNA on-target activity in the psiCHECK system.

Table 8. On-target activity of siRNAs at 9 concentration points using psiCHECK

| Remaining percentage of target gene mRNA expression in psi-check (mean) | | | | | |
|---|---|---|---|---|---|
| Double strand code | 20.0000 nM | 6.6667 nM | 2.2222 nM | 0.7407 nM | 0.2469 nM |
| TJR103295 | 14.22 | 16.13 | 15.25 | 15.47 | 15.61 |
| TJR103296 | 11.52 | 11.37 | 11.25 | 11.53 | 12.58 |
| TJR103298 | 5.83 | 5.38 | 5.38 | 5.61 | 7.55 |
| TJR103299 | 7.58 | 8.17 | 8.32 | 10.16 | 11.52 |
| TJR103300 | 6.14 | 7.08 | 7.95 | 6.83 | 10.04 |
| TJR103301 | 7.78 | 7.19 | 8.28 | 9.01 | 10.12 |
| TJR103302 | 12.37 | 12.17 | 11.40 | 12.43 | 19.56 |
| TJR103303 | 7.64 | 7.48 | 9.46 | 9.08 | 9.57 |
| TJR103304 | 14.83 | 14.17 | 15.25 | 15.74 | 16.13 |
| TJR103305 | NA | 17.60 | 14.02 | 16.49 | 15.97 |
| TJR103306 | NA | 9.76 | 11.77 | 10.91 | 12.68 |
| TJR103307 | 11.75 | 7.75 | 8.61 | 8.03 | 6.92 |
| TJR103308 | NA | 18.75 | 12.21 | 11.34 | 11.47 |
| TJR103309 | 11.11 | 6.94 | 6.74 | 8.41 | 9.84 |
| TJR103310 | 20.80 | 14.36 | 11.44 | 10.59 | 11.03 |
| TJR103311 | 39.25 | 15.55 | 10.75 | 11.87 | 13.47 |
| Double strand code | 0.0823 nM | 0.0274 nM | 0.0091 nM | 0.0030 nM | IC50 (nM) |
| TJR103295 | 15.70 | 19.28 | 34.57 | 59.56 | 0.005 |
| TJR103296 | 15.29 | 18.78 | 35.45 | 59.82 | 0.005 |
| TJR103298 | 13.91 | 24.12 | 50.96 | 73.18 | 0.009 |

(continued)

| Remaining percentage of target gene mRNA expression in psi-check (mean) | | | | | |
|---|---|---|---|---|---|
| TJR103299 | 14.77 | 26.03 | 49.70 | 76.61 | 0.009 |
| TJR103300 | 15.28 | 30.70 | 64.18 | 86.05 | 0.014 |
| TJR103301 | 10.89 | 16.81 | 29.63 | 56.44 | 0.004 |
| TJR103302 | 14.56 | 18.04 | 37.29 | 61.37 | 0.005 |
| TJR103303 | 14.06 | 32.60 | 64.04 | 85.63 | 0.015 |
| TJR103304 | 21.26 | 28.36 | 53.68 | 84.00 | 0.010 |
| TJR103305 | 18.09 | 38.92 | 72.66 | 100.48 | 0.017 |
| TJR103306 | 17.12 | 39.94 | 76.43 | 89.56 | 0.021 |
| TJR103307 | 14.34 | 35.44 | 73.03 | 89.82 | 0.018 |
| TJR103308 | 15.87 | 33.34 | 70.15 | 98.44 | 0.016 |
| TJR103309 | 14.33 | 28.80 | 70.19 | 96.40 | 0.015 |
| TJR103310 | 13.55 | 34.03 | 69.74 | 92.91 | 0.016 |
| TJR103311 | 22.85 | 46.03 | 87.48 | 93.19 | 0.026 |

**Example 10. On-Target Activity of siRNA Conjugates at 2 Endogenous Concentration Points**

The siRNA conjugates were subjected to an *in vitro* molecular-level activity screening in HCC38 cells using 2 concentration gradients.

HCC38 cells were cultured at 37 °C with 5% $CO_2$ in a 1640 culture medium containing 10% fetal bovine serum. 24 h prior to transfection, the HCC38 cells were seeded into a 96-well plate at a density of $1.5 \times 10^4$ cells per well, with each well containing 100 μL of the culture medium.

The cells were transfected with the siRNA conjugates using Lipofectamine RNAi Max (Invitrogen, 13778-150) according to the instructions. 0.3 μL of Lipofectamine RNAi Max was used for each well. A total of 2 concentration points were set for the siRNA conjugates, i.e., 10 nM and 2 nM, respectively. 24 h after transfection, RNA was extracted for Q-PCR to detect the activity of the siRNA conjugates. The results are shown in Table 9. The siRNA conjugates of the present disclosure exhibit good MMP7 mRNA on-target activity in HCC38 cells.

Table 9. Activity results of siRNA conjugates at 2 concentration points at the HCC38 cellular level

| Conjugate code | 10 nM remaining expression (%) | 2 nM remaining expression (%) |
|---|---|---|
| TJR103312 | 8.20 | 11.35 |
| TJR103313 | 4.23 | 8.05 |
| TJR103314 | 8.03 | 14.73 |

## Claims

**1.** An siRNA, comprising a sense strand and an antisense strand forming a double-stranded region, wherein

the sense strand comprises at least 15 contiguous nucleotides and differs from any one of the nucleotide sequences of SEQ NO: 1 to SEQ ID NO: 10 by no more than 3 nucleotides; and
the antisense strand comprises at least 15 contiguous nucleotides and differs from any one of the nucleotide sequences of SEQ ID NO: 31 to SEQ ID NO: 40 by no more than 3 nucleotides.

**2.** The siRNA according to claim 1, wherein:

the sense strand comprises at least 17 contiguous nucleotides differing from any one of the nucleotide sequences of SEQ NO: 1 to SEQ ID NO: 10 by no more than 3 nucleotides;

the antisense strand comprises at least 17 contiguous nucleotides differing from any one of the nucleotide sequences of SEQ ID NO: 31 to SEQ ID NO: 40 by no more than 3 nucleotides;
preferably, the sense strand comprises at least 19 contiguous nucleotides differing from any one of the nucleotide sequences of SEQ ID NO: 1 to SEQ ID NO: 10 by no more than 3 nucleotides; preferably, the nucleotide sequence difference is no more than 1 nucleotide, and/or
the antisense strand comprises at least 21 contiguous nucleotides differing from any one of the nucleotide sequences of SEQ ID NO: 31 to SEQ ID NO: 40 by no more than 3 nucleotides; preferably, the nucleotide sequence difference is no more than 1 nucleotide.

**3.** The siRNA according to claim 1 or 2, comprising or selected from any one of the following groups:

group 1), a sense strand set forth in SEQ ID NO: 1 and an antisense strand set forth in SEQ ID NO: 31;
group 2), a sense strand set forth in SEQ ID NO: 2 and an antisense strand set forth in SEQ ID NO: 32;
group 3), a sense strand set forth in SEQ ID NO: 3 and an antisense strand set forth in SEQ ID NO: 33;
group 4), a sense strand set forth in SEQ ID NO: 4 and an antisense strand set forth in SEQ ID NO: 34;
group 5), a sense strand set forth in SEQ ID NO: 5 and an antisense strand set forth in SEQ ID NO: 35;
group 6), a sense strand set forth in SEQ ID NO: 6 and an antisense strand set forth in SEQ ID NO: 36;
group 7), a sense strand set forth in SEQ ID NO: 7 and an antisense strand set forth in SEQ ID NO: 37;
group 8), a sense strand set forth in SEQ ID NO: 8 and an antisense strand set forth in SEQ ID NO: 38;
group 9), a sense strand set forth in SEQ ID NO: 9 and an antisense strand set forth in SEQ ID NO: 39; and
group 10), a sense strand set forth in SEQ ID NO: 10 and an antisense strand set forth in SEQ ID NO: 40.

**4.** The siRNA according to any one of claims 1-3, wherein at least one nucleotide in the sense strand and/or the antisense strand is a modified nucleotide.

**5.** The siRNA according to claim 4, wherein:

three contiguous nucleotides in the sense strand are 2'-fluoro-modified nucleotides; and/or
in the direction from the 5' end to the 3' end, nucleotides at positions 2, 6, 12, 14, and 16 of the antisense strand are each independently a 2'-fluoro-modified nucleotide;
preferably, the nucleotides at positions 2, 6, 12, 14, and 16 of the antisense strand are each independently a 2'-fluoro-modified nucleotide, or a nucleotide at position 2, 4, 6, 10, 12, 14, 16, or 18 of the antisense strand is each independently a 2'-fluoro-modified nucleotide;
preferably, in the direction from the 5' end to the 3' end, nucleotides at positions 7, 8, and 9 of the sense strand are 2'-fluoro-modified nucleotides;
nucleotides at the remaining positions in the sense strand and the antisense strand are 2-methoxy-modified nucleotides.

**6.** The siRNA according to any one of claims 1-5, wherein at least one phosphodiester group in the sense strand and/or the antisense strand is a phosphodiester group with a modification group, preferably a phosphorothioate diester group.

**7.** The siRNA according to claim 6, wherein the phosphodiester group with a modification group is present at one or more of the following positions selected from the group consisting of:

a position between the 1st and 2nd nucleotides at the 5' end of the sense strand;
a position between the 2nd and 3rd nucleotides at the 5' end of the sense strand;
a position between the 1st and 2nd nucleotides at the 5' end of the antisense strand;
a position between the 2nd and 3rd nucleotides at the 5' end of the antisense strand;
a position between the 1st and 2nd nucleotides at the 3' end of the antisense strand;
a position between the 2nd and 3rd nucleotides at the 3' end of the antisense strand;
preferably, the sense strand and/or the antisense strand comprise a plurality of phosphodiester groups with a modification group.

**8.** The siRNA according to any one of claims 1-7, wherein

the sense strand comprises the nucleotide sequence set forth in any one of SEQ ID NO: 11 to SEQ ID NO: 27; and/or

the antisense strand comprises the nucleotide sequence set forth in any one of SEQ ID NO: 41 to SEQ ID NO: 57.

**9.** An siRNA conjugate, comprising:

the siRNA according to any one of claims 1-8, and
a targeting ligand linked to the siRNA, wherein
the targeting ligand has affinity for a cellular receptor expressed on an epithelial cell;
preferably, the targeting ligand comprises an integrin-targeting ligand;
more preferably, the targeting ligand comprises an αvβ6 integrin-targeting ligand;
further preferably, the targeting ligand has a structure represented by I-14:

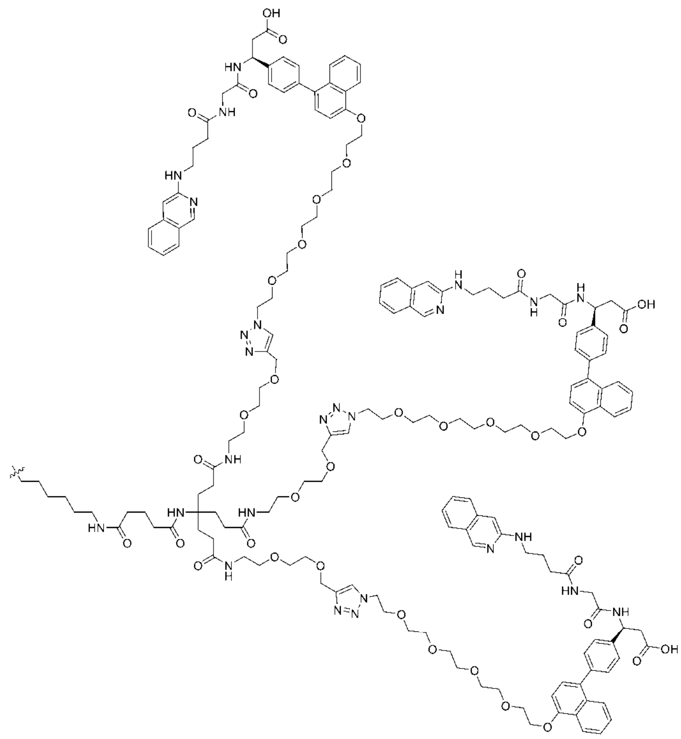

I-14.

**10.** The siRNA conjugate according to claim 9, wherein

the targeting ligand is linked to the sense strand of the siRNA;
preferably, the targeting ligand is linked to the 5' end of the sense strand of the siRNA.

**11.** The siRNA conjugate according to claim 9 or 10, wherein

the sense strand comprises the nucleotide sequence set forth in any one of SEQ ID NO: 28 to SEQ ID NO: 30; and/or
the antisense strand comprises the nucleotide sequence set forth in any one of SEQ ID NO: 58 to SEQ ID NO: 60.

**12.** A pharmaceutical composition, comprising:
the siRNA according to any one of claims 1-8 or the siRNA conjugate according to any one of claims 9-11, and a pharmaceutically acceptable carrier.

**13.** A cell, comprising:

the siRNA according to any one of claims 1-8, or
the siRNA conjugate according to any one of claims 9-11.

**14.** A kit, comprising:

the siRNA according to any one of claims 1-8, or
the siRNA conjugate according to any one of claims 9-11, or
the pharmaceutical composition according to claim 12.

**15.** A method for reducing the expression of matrix metalloproteinase 7 (MMP7), comprising:
administering to a subject the siRNA according to any one of claims 1-8, or the siRNA conjugate according to any one of claims 9-11, or the pharmaceutical composition according to claim 12.

**16.** A method for treating and/or preventing a disease, comprising:

administering to a subject the siRNA according to any one of claims 1-8, or the siRNA conjugate according to any one of claims 9-11, or the pharmaceutical composition according to claim 12, wherein
preferably, the disease is selected from the group consisting of asthma, fibrosis, chronic inflammation, interstitial lung disease, infectious disease, acute lung injury, pulmonary hypertension, and cancer;
preferably, the infectious disease is SARS-COV-2;
preferably, the acute lung injury is acute respiratory distress syndrome;
preferably, the fibrosis is idiopathic pulmonary fibrosis, renal fibrosis, or liver fibrosis.

**17.** A method for delivering an siRNA that inhibits the expression and/or replication of MMP7 *in vivo,* comprising:

administering to a subject the siRNA according to any one of claims 1-8, or the siRNA conjugate according to any one of claims 9-11, or the pharmaceutical composition according to claim 12, wherein
preferably, the siRNA is delivered extrahepatically;
more preferably, the siRNA is delivered to the lung.

**18.** A method for preparing an siRNA or an siRNA conjugate, comprising:
synthesizing the siRNA according to any one of claims 1-8 or the siRNA conjugate according to any one of claims 9-11.

## INTERNATIONAL SEARCH REPORT

International application No.
**PCT/CN2024/098635**

### A. CLASSIFICATION OF SUBJECT MATTER

C12N15/113(2010.01)i; A61K31/713(2006.01)i; A61K31/712(2006.01)i; A61K31/7125(2006.01)i; A61K38/00(2006.01)i; A61P11/00(2006.01)i; A61P35/00(2006.01)i

According to International Patent Classification (IPC) or to both national classification and IPC

### B. FIELDS SEARCHED

Minimum documentation searched (classification system followed by classification symbols)

IPC: C12N A61K A61P

Documentation searched other than minimum documentation to the extent that such documents are included in the fields searched

Electronic data base consulted during the international search (name of data base and, where practicable, search terms used)

CNABS, CNTXT, ENTXT, ENTXTC, WOTXT, USTXT, EPTXT, CNKI, 万方, WANFANG, PUBMED, WEB OF SCIENCE, BING, STN, GENBANK, EMBL, 中国专利生物序列检索系统, China Patent Biological Sequence Search System, MMP-7, 基质金属蛋白酶7, matrix metalloproteinase 7, 基质金属肽酶7, 基质溶解素, 修饰, 甲氧基, 氟代, 硫代磷酸二酯键, 反义寡核苷酸, siRNA, RNAi, 干扰RNA, 正义链, 反义链, 有义链, AON, antisense, oligonucleotides, 靶向配体, 缀合物, 整联蛋白, 整合素, integrin, conjugate, I-14, SEQ ID NOs. 1-57

### C. DOCUMENTS CONSIDERED TO BE RELEVANT

| Category* | Citation of document, with indication, where appropriate, of the relevant passages | Relevant to claim No. |
|---|---|---|
| X | WO 2023070082 A2 (ARROWHEAD PHARMACEUTICALS, INC.) 27 April 2023 (2023-04-27)<br>claims 1-60, and description, paragraphs 8-23, 73 and 173, tables 1-10, and embodiments 2-25 | 1-18 |
| X | WO 2006092795 A2 (QBI ENTPRPRISES LTD.) 08 September 2006 (2006-09-08)<br>description, pages 14, 18-22, 27-28 and 84-88, and tables I-J | 1-8, 12-18 |
| Y | WO 2006092795 A2 (QBI ENTPRPRISES LTD.) 08 September 2006 (2006-09-08)<br>description, pages 14, 18-22, 27-28 and 84-88, and tables I-J | 9-18 |
| Y | CN 111526880 A (ARROWHEAD PHARMACEUTICALS, INC.) 11 August 2020 (2020-08-11)<br>claims 1-36 | 9-18 |

☑ Further documents are listed in the continuation of Box C. ☑ See patent family annex.

* Special categories of cited documents:
- "A" document defining the general state of the art which is not considered to be of particular relevance
- "D" document cited by the applicant in the international application
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| **12 September 2024** | **20 September 2024** |
| Name and mailing address of the ISA/CN<br>**China National Intellectual Property Administration (ISA/CN)**<br>**China No. 6, Xitucheng Road, Jimenqiao, Haidian District, Beijing 100088** | Authorized officer<br><br>Telephone No. |

Form PCT/ISA/210 (second sheet) (July 2022)

**INTERNATIONAL SEARCH REPORT**

International application No.
**PCT/CN2024/098635**

**C. DOCUMENTS CONSIDERED TO BE RELEVANT**

| Category* | Citation of document, with indication, where appropriate, of the relevant passages | Relevant to claim No. |
|---|---|---|
| Y | CN 110832077 A (ARROWHEAD PHARMACEUTICALS, INC.) 21 February 2020 (2020-02-21)<br>description, table 6, and figures 6 and 10 | 9-18 |
| A | YUAN, T. et al. "Silencing MMP7 Expression with a Lung-targeted RNAimolecule Limits Fibrosis and Preserves Pulmonaryfunction in Bleomycin-injured Rats"<br>*European Respiratory Journal,* Vol. vol. Suppl.66, 31 December 2022 (2022-12-31),<br>page 864 | 1-18 |
| A | LIAO, C.H. et al. "Homo Sapiens Matrix Metallopeptidase 7 (MMP7), mRNA"<br>*Genbank Accession: NM_002423.5,* 12 February 2023 (2023-02-12),<br>pages 1-3 | 1-18 |
| A | ROSAS, I.O. et al. "MMP1 and MMP7 as Potential Peripheral Blood Biomarkers in Idiopathic Pulmonary Fibrosis"<br>*PLoS Medicine,* Vol. 5, No. (4), 29 April 2008 (2008-04-29),<br>pp. 0623-0633 | 1-18 |
| A | 王文婷 等 (WANG, Wenting et al.). "基质金属蛋白酶及整合素在肺纤维化中的作用 (Important Roles of Matrix Metalloproteinases and Integrins in Pulmonary Fibrosis)"<br>*药物生物技术 (Chinese Journal of Pharmaceutical Biotechnology),*<br>Vol. 30, No. (2), 15 April 2023 (2023-04-15),<br>pp. 180-185 | 1-18 |
| A | 李玉红 等 (LI, Yuhong et al.). "基质金属蛋白酶-7在肝胆疾病中的作用与意义 (The Role and Significance of Matrix Metalloproteinase-7 in Hepatobiliary Diseases)"<br>*国际儿科学杂志 (International Journal of Pediatrics),*<br>Vol. 49, No. (1), 31 January 2022 (2022-01-31),<br>pp. 48-51 | 1-18 |
| A | 杨波 等 (YANG, Bo et al.). "MMP7反义寡核苷酸对肺腺癌A549细胞粘附和侵袭能力的影响 (Effects of MMP7 Antisense Oligodeoxynucleotide on Adhesion and Invasion of Human Lung Adenocarcinoma Cell A549)"<br>*重庆医科大学学报 (Journal of Chongqing Medical University),*<br>Vol. 35, No. (8), 31 December 2010 (2010-12-31),<br>pp. 1160-1163 | 1-18 |

Form PCT/ISA/210 (second sheet) (July 2022)

**INTERNATIONAL SEARCH REPORT**

International application No.
**PCT/CN2024/098635**

**Box No. I Nucleotide and/or amino acid sequence(s) (Continuation of item 1.c of the first sheet)**

1. With regard to any nucleotide and/or amino acid sequence disclosed in the international application, the international search was carried out on the basis of a sequence listing:
   - a. ☑ forming part of the international application as filed.
   - b. ☐ furnished subsequent to the international filing date for the purposes of international search (Rule 13*ter*.1(a)),
     - ☐ accompanied by a statement to the effect that the sequence listing does not go beyond the disclosure in the international application as filed.

2. ☐ With regard to any nucleotide and/or amino acid sequence disclosed in the international application, this report has been established to the extent that a meaningful search could be carried out without a WIPO Standard ST.26 compliant sequence listing.

3. Additional comments:

Form PCT/ISA/210 (continuation of first sheet) (July 2022)

**INTERNATIONAL SEARCH REPORT**

International application No.
**PCT/CN2024/098635**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **16-17**
because they relate to subject matter not required to be searched by this Authority, namely:

Claim 16 relates to a method for treating and/or preventing diseases, and claim 17 relates to a method for in vivo delivery for inhibition of MMP7 expression and/or replication, that is, claims 16-17 relate to subject matter for which no search is required by the International Searching Authority as defined in PCT Rule 39.1: (4) methods for treatment of the human or animal body by surgery or therapy, as well as diagnostic methods. The international search is made on the basis of the use of siRNA or an siRNA conjugate in the preparation of a drug for treating and/or preventing diseases, or inhibiting MMP7 expression and/or replication.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (July 2022)

**INTERNATIONAL SEARCH REPORT**
**Information on patent family members**

International application No.
**PCT/CN2024/098635**

| Patent document cited in search report | Publication date (day/month/year) | Patent family member(s) | Publication date (day/month/year) |
|---|---|---|---|
| WO 2023070082 A2 | 27 April 2023 | AU 2022368927 A1 | 11 April 2024 |
| | | TW 202334416 A | 01 September 2023 |
| | | EP 4419689 A2 | 28 August 2024 |
| | | WO 2023070082 A3 | 06 July 2023 |
| | | US 2023265437 A1 | 24 August 2023 |
| | | KR 20240099296 A | 28 June 2024 |
| | | IL 312239 A | 01 June 2024 |
| | | CO 2024005110 A2 | 30 May 2024 |
| | | CA 3235224 A1 | 27 April 2023 |
| WO 2006092795 A2 | 08 September 2006 | WO 2006092795 A3 | 01 March 2007 |
| CN 111526880 A | 11 August 2020 | MX 2023007368 A | 04 July 2023 |
| | | IL 274300 A | 30 June 2020 |
| | | IL 274300 B1 | 01 May 2024 |
| | | TN 2020000059 A1 | 06 January 2022 |
| | | UA 128250 C2 | 22 May 2024 |
| | | TW 202017570 A | 16 May 2020 |
| | | TWI 825039 B | 11 December 2023 |
| | | US 2020369613 A1 | 26 November 2020 |
| | | US 11597701 B2 | 07 March 2023 |
| | | US 2024076270 A1 | 07 March 2024 |
| | | CL 2020001125 A1 | 28 August 2020 |
| | | ECSP 20028090 A | 31 July 2020 |
| | | PH 12020550263 A1 | 01 March 2021 |
| | | SG 11202002967 SA | 28 May 2020 |
| | | WO 2019089765 A1 | 09 May 2019 |
| | | EP 3703700 A1 | 09 September 2020 |
| | | EP 3703700 A4 | 04 August 2021 |
| | | JOP 20200102 A1 | 30 October 2022 |
| | | JP 2023073520 A | 25 May 2023 |
| | | BR 112020006901 A2 | 13 October 2020 |
| | | JP 2021501754 A | 21 January 2021 |
| | | JP 7445594 B2 | 07 March 2024 |
| | | CA 3079402 A1 | 09 May 2019 |
| | | MX 2020004554 A | 13 August 2020 |
| | | KR 20200083523 A | 08 July 2020 |
| | | CR 20200178 A | 28 June 2020 |
| | | AU 2018359515 A1 | 18 June 2020 |
| CN 110832077 A | 21 February 2020 | PH 12019502660 A1 | 08 June 2020 |
| | | TN 2019000308 A1 | 07 May 2021 |
| | | ZA 202100643 B | 29 March 2023 |
| | | EP 3649240 A1 | 13 May 2020 |
| | | EP 3649240 A4 | 07 July 2021 |
| | | UY 37800 A | 02 January 2019 |
| | | US 2019010494 A1 | 10 January 2019 |
| | | US 10590416 B2 | 17 March 2020 |
| | | AU 2018297262 A1 | 27 February 2020 |
| | | JOP 20190263 A1 | 12 November 2019 |
| | | CA 3061752 A1 | 10 January 2019 |
| | | KR 20200024793 A | 09 March 2020 |
| | | CR 20190572 A | 23 May 2020 |

Form PCT/ISA/210 (patent family annex) (July 2022)

**INTERNATIONAL SEARCH REPORT**
**Information on patent family members**

International application No.
**PCT/CN2024/098635**

| Patent document cited in search report | Publication date (day/month/year) | Patent family member(s) | Publication date (day/month/year) |
|---|---|---|---|
| | | WO 2019010274 A1 | 10 January 2019 |
| | | JP 2023158192 A | 26 October 2023 |
| | | CO 2019014671 A2 | 17 January 2020 |
| | | ECSP 20000655 A | 29 May 2020 |
| | | CL 2020000019 A1 | 12 June 2020 |
| | | MX 2019014800 A | 10 February 2020 |
| | | BR 112019023650 A2 | 02 June 2020 |
| | | PE 20200746 A1 | 24 July 2020 |
| | | IL 271843 A | 27 February 2020 |
| | | TW 201919654 A | 01 June 2019 |
| | | JP 2020526192 A | 31 August 2020 |
| | | US 2020299691 A1 | 24 September 2020 |
| | | US 11214802 B2 | 04 January 2022 |
| | | US 2022090077 A1 | 24 March 2022 |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- CN 202310696482
- CN 202410074824
- WO 2022028462 A1
- WO 2023274395 A1
- WO 2023208023 A1
- WO 2019089765 A1
- WO 2019161213 A1

### Non-patent literature cited in the description

- Antibodies: A Laboratory Manual and Molecular Cloning: A Laboratory Manual. Cold Spring Harbor Laboratory